(19) Europäisches Patentamt
European Patent Office
Office européen des brevets

(11) **EP 3 189 223 B1**

(12) **EUROPÄISCHE PATENTSCHRIFT**

(45) Veröffentlichungstag und Bekanntmachung des Hinweises auf die Patenterteilung:
**27.06.2018 Patentblatt 2018/26**

(21) Anmeldenummer: **15760433.1**

(22) Anmeldetag: **02.09.2015**

(51) Int Cl.:
*F02C 6/00* (2006.01)          *B01J 8/06* (2006.01)
*B01J 19/00* (2006.01)

(86) Internationale Anmeldenummer:
**PCT/EP2015/070090**

(87) Internationale Veröffentlichungsnummer:
**WO 2016/034647 (10.03.2016 Gazette 2016/10)**

(54) **VERFAHREN UND ANLAGE ZUR PARALLELEN ERZEUGUNG VON MECHANISCHER LEISTUNG UND HERSTELLUNG VON KOHLENWASSERSTOFFEN**

METHOD AND SYSTEM FOR GENERATING A MECHANICAL OUTPUT AND PRODUCING HYDROCARBONS IN A PARALLEL MANNER

PROCÉDÉ ET INSTALLATION DE GÉNÉRATION D'ÉNERGIE MÉCANIQUE ET DE PRODUCTION D'HYDROCARBURES EN PARALLÈLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2014 EP 14183742**

(43) Veröffentlichungstag der Anmeldung:
**12.07.2017 Patentblatt 2017/28**

(73) Patentinhaber: **Linde AG**
**80331 München (DE)**

(72) Erfinder: **BRUDER, David**
**82379 München (DE)**

(56) Entgegenhaltungen:
**FR-A- 1 445 870      GB-A- 2 148 734
GB-A- 2 296 719      US-A- 5 048 284**

EP 3 189 223 B1

**Beschreibung**

Die Erfindung betrifft ein Verfahren und eine Anlage zur parallelen Erzeugung von mechanischer Leistung und Herstellung von Kohlenwasserstoffen gemäß den Oberbegriffen der unabhängigen Patentansprüche.

Stand der Technik

Bei einer Vielzahl von Verfahren zur Herstellung chemischer Reaktionsprodukte werden Reaktoren eingesetzt, die durch Brenner beheizte Reaktionsrohre umfassen, durch welche ein Einsatz geführt und dabei zumindest teilweise zu den gewünschten Reaktionsprodukten umgesetzt wird. Beispiele für entsprechende Verfahren sind das Dampfspalten (Steamcracking), die Dehydrierung von Alkanen oder auch die Synthesegas- oder Ammoniakerzeugung.

Entsprechende Verfahren und Vorrichtungen sind umfangreich in der Literatur beschrieben. Zu Verfahren und Vorrichtungen zum Dampfspalten sei beispielsweise auf den Artikel "Ethylene" in Ullmann's Encyclopedia of Industrial Chemistry, online seit 15. April 2007, DOI 10.1002/14356007.a10_045.pub2, hingewiesen. Verfahren und Vorrichtungen zur Dehydrierung von Alkanen, insbesondere von Propan zu Propylen und von Isobutan zu Isobuten, gehen beispielsweise aus dem Artikel "Propene" in Ullmann's Encyclopedia of Industrial Chemistry, Onlineausgabe, 15. Juni 2000, DOI 10.1002/14356007.a22_211, Abschnitt 4.3., "Propane Dehydrogenation", hervor.

Seit längerem besteht der Wunsch, entsprechende Reaktoren mit Vorrichtungen zur Erzeugung von mechanischer Leistung zu koppeln. Letztere können beispielsweise unter Verwendung von Verbrennungskraftmaschinen, insbesondere von Gasturbinen, realisiert sein. Als Vorbild für entsprechende kombinierte Verfahren dienen dabei bekannte Gas-und-Dampf-Verfahren bzw. entsprechende Anlagen.

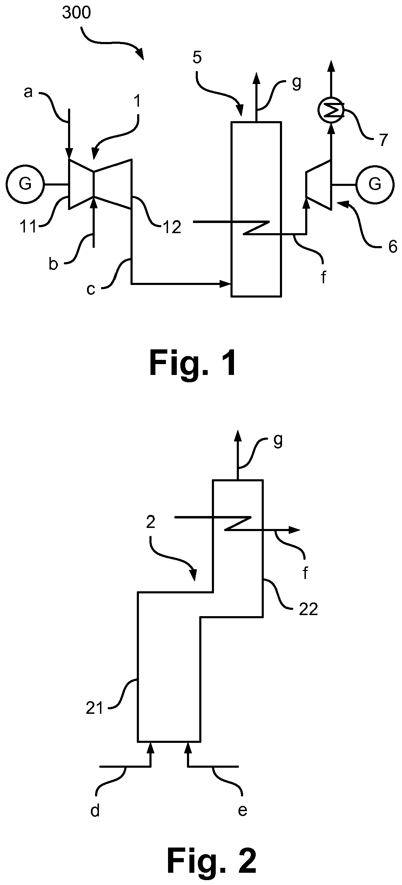

In einem Gas-und-Dampf-Verfahren, wie es auch in Figur 1 veranschaulicht und unten erläutert ist, wird mittels einer Gasturbine ein Sauerstoff enthaltendes Verbrennungsunterstützungsgas, typischerweise Luft, angesaugt und verdichtet. In eine Brennkammer der Gasturbine wird ein geeigneter Brennstoff, typischerweise Erdgas oder ein anderes Gasgemisch, eingebracht und unter Druck in der durch das Verbrennungsunterstützungsgas gebildeten Atmosphäre verbrannt. Durch eine Entspannung des dabei gebildeten Verbrennungsabgases (auch als Heißgas bezeichnet) wird eine Expansionsstufe der Gasturbine und über diese ein mit der Gasturbine gekoppelter Generator angetrieben.

In dem Verbrennungsabgas stromab der Gasturbine noch enthaltene Wärme kann in einem Abhitzedampferzeuger (sogenannter Heat Recovery Steam Generator, HRSG) zur Erzeugung von Druckdampf verwendet werden. Mittels des Druckdampfs kann eine Dampfturbine angetrieben werden. Die Leistung der Dampfturbine wird typischerweise zur weiteren Erzeugung von elektrischer Energie genutzt, und zwar in dem mit der Gasturbine gekoppelten oder einem weiteren Generator.

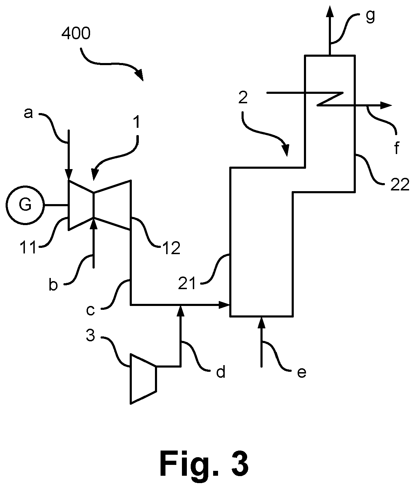

Kombinierte Verfahren, bei denen Gasturbinen und die zuvor erläuterten beheizten Reaktoren zum Einsatz kommen, sind grundsätzlich ebenfalls bekannt, wie zu Figur 3 erläutert. Wie unten im Detail ausgeführt, kommt es jedoch in derartigen Anlagen zu einer deutlichen Verringerung des Strahlungszonenwirkungsgrads des verwendeten Reaktors. Die Gesamteffizienz entsprechender Anlagen liegt daher allenfalls geringfügig über der separater Anlagen zur Erzeugung von elektrischer Energie und zur Gewinnung von Kohlenwasserstoffen. Der geringe Effizienzvorteil kombinierter Anlagen rechtfertigt daher den Aufwand der Kopplung normalerweise nicht.

Insbesondere ergibt sich in entsprechenden Anlagen eine Abhängigkeit im Betrieb des beheizten Reaktors von dem Betrieb der Gasturbine. Fällt letztere aus, muss auch der Reaktor im Extremfall stillgelegt werden, was zu entsprechend kostenintensiven Produktionsausfällen führt. Typischerweise können die genannten Reaktoren für einen Dauerbetrieb über mehrere Jahre ausgelegt werden oder sie werden in Form mehrerer paralleler Einheiten ausgeführt, die abwechselnd gewartet oder regeneriert werden. Bei Dampfspaltverfahren können beispielsweise stets fünf bis zehn Reaktoren im Betrieb sein, einer befindet sich im sogenannten Entkokungsbetrieb. Eine Gasturbine bedarf jedoch deutlich häufigerer Wartung.

In der GB 2148734 A ist ein Kraftwerk mit einem Hochgeschwindigkeits-Wirbelschichtverbrennungsreaktor offenbart. Es ist ein unterteiltes Wärmeübertragungs-Wirbelschichtbett bereitgestellt, das dafür vorgesehen ist, heiße Asche, die durch den Wirbelschichtverbrennungsreaktor erzeugt wird, zu verwirbeln und Wärme aus diesem zu extrahieren. Dabei sind Mittel bereitgestellt, die dafür vorgesehen sind, die jeweiligen Anteile der heißen Asche, die durch die Unterteilung gebildeten Abschnitte des Wärmeübertragungsbetts zirkuliert werden, zu steuern, so dass die jeweiligen Leistungsgewinne der Abschnitte gesteuert werden können. Ein Abschnitt des unterteilten Wirbelschichtbetts kann Prozessdampf erzeugen und der andere Abschnitt kann heiße Arbeitsluft für eine Turbine bereitstellen.

Aus der US 5,048,284 A und der GB 2 296 719 A sind kombinierte Verfahren bekannt, bei denen eine autotherme Reformierung vorgenommen und eine Turbine betrieben wird. Aus der FR 1 445 870 A ist ein Reformierreaktor bekannt, der in Kombination mit einer Turbine betrieben wird.

Aufgabe vorliegender Erfindung ist es daher, kombinierte Verfahren zur Erzeugung von elektrischer Energie und zur Herstellung von Kohlenwasserstoffen insbesondere in ihrer Effizienz zu verbessern.

Offenbarung der Erfindung

Diese Aufgabe wird durch ein Verfahren und eine Anlage zur Erzeugung von mechanischer Leistung und zur Herstellung von Kohlenwasserstoffen mit den Merkmalen der jeweiligen unabhängigen Patentansprüche gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Im Folgenden wird häufig auf Wirkungsgrade von thermischen Prozessen Bezug genommen; es gelten hierbei folgende Definitionen:

Der feuerungstechnische ("thermische") Wirkungsgrad (FTW, ny_FTW) bezeichnet den Anteil der eingebrachten Wärmeleistung (P_zugeführt), die nicht über das Verbrennungsabgas (P_Abgas) an die Umgebung verloren geht:

$$ny\_FTW = 1 - P\_Abgas / P\_zugeführt$$

Nicht berücksichtigt werden hier die Verluste an die Umgebung durch Wärmeleitung heißer Bauteile, da diese typischerweise deutlich kleiner als die Abgasverluste sind.

Der Strahlungszonenwirkungsgrad (SZW, ny_SZ) bezeichnet den Anteil der eingebrachten Wärmeleistung (P_zugeführt), der in einem Feuerungsraum an ein Prozessmedium indirekt übertragen wird (P_Prozess):

$$ny\_SZ = P\_Prozess / P\_zugeführt$$

Die Übertragung erfolgt dabei typischerweise bei Temperaturen deutlich über 1.000 °C und vorzugsweise durch Strahlung. Typische Strahlungszonenwirkungsgrade von ausschließlich direkt, d.h. nur mittels Brennern, nicht jedoch z.B. mittels vorgewärmter Verbrennungsluft, beheizten Reaktoren zum Dampfspalten betragen ca. 0,42 (42%).

Der elektrische Wirkungsgrad (ny_el) bezeichnet den Anteil der eingebrachten Wärmeleistung (P_zugeführt) eines Wärmekraftprozesses, der als Nettoleistung in Form elektrischer Leistung abgegeben wird (die Nettoleistung bezeichnet die Leistung des Wärmekraftprozesses abzüglich der für Nebenaggregate wie Pumpen und Verdichter benötigten Leistung):

$$ny\_el = P\_el / P\_zugeführt$$

Der Begriff der "energetischen Effizienz" wird hier allgemein als Vergleichsbegriff verwendet, der hier die benötigte Heizleistung unterschiedlicher Verfahren oder kombinierter Verfahren zur Herstellung einer bestimmten Menge eines oder mehrerer Produkte bzw. zur Erzeugung einer bestimmten Menge elektrischer Leistung bewertet. Der Begriff wird beispielsweise für die Erzeugung elektrischer Leistung über einen einstufigen Dampfprozess, eine Gasturbine und ein kombiniertes Gas-und Dampf-Verfahren verwendet. Typischerweise steigt hier die Effizienz in der angegebenen Reihenfolge, d.h. die für eine bestimmte Menge erzeugten elektrischen Stroms eingesetzte Heizleistung sinkt.

Die Feuerungsleistung eines Brennstoffes wird im Rahmen dieser Anmeldung typischerweise auf den unteren Heizwert (Hu) bezogen. Es handelt sich um die bei einer Verbrennung maximal nutzbare Wärmemenge, bei der es nicht zu einer Kondensation des im Abgas enthaltenen Wasserdampfs kommt, bezogen auf die Menge des eingesetzten Brennstoffs.

Der gängige Sprachgebrauch bezeichnet mit dem Begriff "Gasturbine" eine Anordnung, die, wie eingangs erwähnt, eine Verdichterstufe, die Expansionsstufe als eigentliche Gasturbine sowie eine zwischen Verdichterstufe und Expansionsstufe geschaltete Brennkammer umfasst. Der Brennkammer wird über die Verdichterstufe ein komprimiertes Verbrennungsunterstützungsgas, beispielsweise Luft, zugeführt. Über einen Brennstoffeinlass gelangt der (in der Regel flüssige oder gasförmige) Brennstoff in die Brennkammer. Der Brennstoff wird in der Brennkammer mit dem Gasgemisch zu einem Verbrennungsabgas, dem sogenannten Heißgas, verbrannt. Das Heißgas wird in der Expansionsstufe entspannt, wobei thermische in mechanische Leistung gewandelt wird. Die mechanische Leistung wird über eine oder mehrere Wellen abgeführt. Ein Teil der mechanischen Leistung wird zum Betrieb der Verdichterstufe verwendet, der verbleibende Anteil dient beispielsweise zum Antrieb eines Generators. Nach der Entspannung wird das Verbrennungsabgas als Abgas abgeblasen oder, wie vorliegend, als Wärmemedium eingesetzt.

Im Rahmen dieser Anmeldung wird der Begriff "Verbrennungsunterstützungsgas" verwendet, um zum Ausdruck zu bringen, dass eine Verbrennung eines Brennstoffs nicht zwingend mit Luft ("Verbrennungsluft") erfolgen muss, sondern auch in einem anderen, jedoch zwingend sauerstoffhaltigen, Gasgemisch erfolgen kann:

Wie auch im Fall von Gasturbinen wird befeuerten Reaktoren, wie sie eingangs erwähnt wurden, neben dem Brennstoff, der in entsprechenden Brennern zur Unterfeuerung verbrannt wird, ein Verbrennungsunterstützungsgas zugeführt. Als Verbrennungsunterstützungsgas wird typischerweise Luft verwendet. Es kann jedoch auch vorgesehen sein, als Verbrennungsunterstützungsgas zumindest teilweise das Verbrennungsabgas einer Gasturbine einzusetzen. Dies ist möglich, weil die Verbrennung eines Brennstoffs in einer Gasturbine typischerweise bei deutlich überstöchiometrischem Sauerstoffangebot erfolgt. In dem Verbrennungsabgas ist daher noch eine beträchtliche Menge an Sauerstoff enthalten, so dass die Verwendung des Verbrennungsabgases als Verbrennungsunterstützungsgas in dem Reaktor möglich ist. Neben einem Verbrennungsabgas einer Gasturbine kann in einem entsprechenden Reaktor auch zusätzliche Luft oder ein sauerstoffhaltiges Gasgemisch zur Regelung der Verbrennung eingesetzt werden. Die sich bei der Verwendung eines Verbrennungsabgases einer Gasturbine als Verbrennungsunterstützungsgas ergebenden Probleme sind unten erläutert und der Ausgangspunkt, an dem die vorliegende Erfindung ansetzt.

Vorteile der Erfindung

Die vorliegende Erfindung geht von einem grundsätzlich bekannten Verfahren zur kombinierten Erzeugung von mechanischer Leistung und Herstellung von Kohlenwasserstoffen aus, bei dem zur Erzeugung der mechanischen Leistung wenigstens eine Verbrennungskraftmaschine unter Erhalt eines Verbrennungsabgases befeuert wird, und bei dem zur Herstellung der Kohlenwasserstoffe wenigstens ein Reaktor unter Verwendung eines Brennstoffs und eines Verbrennungsunterstützungsgases beheizt wird.

Erfindungsgemäß ist dabei vorgesehen, dass zumindest ein Teil des Verbrennungsunterstützungsgases durch indirekten Wärmetausch mit zumindest einem Teil des Verbrennungsabgases der Verbrennungskraftmaschine erwärmt wird. Mit anderen Worten wird gemäß der vorliegenden Erfindung dem Reaktor nicht das Verbrennungsabgas insgesamt zugeführt, sondern allenfalls ein Teil hiervon. Mittels eines anderen Teils oder dem gesamten Verbrennungsabgas wird ein extern zugeführtes Verbrennungsunterstützungsgas, beispielsweise Luft, vorgeheizt.

Ist im Rahmen der vorliegenden Anmeldung dabei davon die Rede, dass ein Brennstoff, ein Verbrennungsunterstützungsgas und/oder ein Verbrennungsabgas einem Reaktor "zugeführt" oder in diesen "eingespeist" wird, bedeutet dies eine Einspeisung in entsprechende Brenner oder eine Brennkammer, nicht in die Reaktionszone, beispielsweise die Reaktionsrohre, eines Reaktors. Ein durch die Reaktionszone, beispielsweise die Reaktionsrohre, geführtes Gasgemisch wird im Rahmen dieser Anmeldung als (rohrseitiges) Prozessgas bezeichnet.

Die vorliegende Erfindung eignet sich insbesondere für Verfahren, bei denen die erzeugte mechanische Leistung zumindest teilweise zum Antreiben eines Generators verwendet, d.h. zumindest teilweise in elektrische Leistung umgewandelt wird. Die Erfindung ist jedoch auch mit Vorteil einsetzbar, wenn mittels der mechanischen Leistung zumindest teilweise wenigstens eine Welle, beispielsweise eines Verdichters und/oder einer Pumpe, angetrieben wird. In diesem Fall kann die angetriebene Einheit beispielsweise Teil des verwendeten Verfahrens zur Herstellung der Kohlenwasserstoffen sein. Beispielsweise kann mit der mechanischen Leistung ein Verdichter zum Verdichten eines Prozessgases oder von Dampf angetrieben werden.

Die vorliegende Erfindung beruht auf der Erkenntnis, dass sich der Wirkungsgrad, genauer der oben definierte Strahlungszonenwirkungsgrad, des Reaktors in entsprechenden kombinierten Verfahren gemäß dem Stand der Technik unter anderem deshalb deutlich reduziert, weil einem dort direkt in den Reaktor zur Unterstützung der Verbrennung des Brennstoffs eingespeisten Verbrennungsabgas einer Gasturbine in bilanzierender Betrachtung ein beträchtlicher Anteil der Energie bereits in der Gasturbine entzogen wurde. Insbesondere kann dies anhand des Sauerstoffgehalts eines entsprechenden Verbrennungsabgases veranschaulicht werden:

Zwangsläufig kommt es in der Gasturbine, selbst wenn dort eine Verbrennung unter deutlich überstöchiometrischem Sauerstoffangebot erfolgt, zu einer Verringerung des Sauerstoffgehalts des Verbrennungsunterstützungsgases. Wird als das Verbrennungsunterstützungsgas in der Gasturbine, wie üblich, Luft mit einem natürlichen Sauerstoffgehalt von ca. 21% verwendet, reduziert sich dieser Sauerstoffgehalt im Verbrennungsabgas auf ca. 14%.

In entsprechenden Reaktoren, insbesondere in Reaktoren, die für das Dampfspalten eingesetzt werden, richtet sich der Strahlungszonenwirkungsgrad jedoch wesentlich nach der Temperatur, die sich durch die Verbrennung des Brennstoffs erzielen lässt und die auf den durch die Reaktionsrohre geführten Einsatz übertragen werden kann. In herkömmlichen Verfahren, d.h. autarken Reaktoren, die nicht mit Gasturbinen gekoppelt sind, wird bei Verwendung von Luft als Verbrennungsunterstützungsgas eine adiabat(isch)e Verbrennungstemperatur von ca. 2.000 °C erreicht. Die adiabatische Verbrennungstemperatur ist diejenige Temperatur, die sich nach dem vollständigen Ablauf einer Verbrennung ergeben würde, wenn das Gasgemisch während der Verbrennung keinerlei Wärme mit der Umgebung austauschen würde. Es handelt sich also um eine theoretische Temperatur, die real nicht erreicht wird, da ein entsprechender Reaktor in der Realität gerade nicht adiabat arbeitet. Bei der adiabatischen Verbrennungstemperatur handelt es sich jedoch um einen fachüblichen Vergleichswert, der die Größe am zweckmäßigsten beschreibt, nach welcher sich der Strahlungszonenwirkungsgrad richtet.

Enthält ein Verbrennungsunterstützungsgas weniger Sauerstoff, weil dieser in einer vorgeschalteten Gasturbine teilweise umgesetzt wurde, sind lediglich noch adiabatische Verbrennungstemperaturen von ca. 1.750 °C erreichbar. Obwohl das Verbrennungsabgas die Gasturbine beispielsweise bei ca. 600 °C verlässt und damit eine beträchtliche Wärmemenge zusätzlich zur Verfügung steht, reicht der verringerte Sauerstoffgehalt dennoch nicht mehr aus, um die adiabatische Verbrennungstemperatur herkömmlicher Reaktoren zu erreichen.

Nimmt man (wiederum in bilanzierender Betrachtung) vereinfacht an, dass die Gasturbine ca. ein Drittel der ihr zugeführten Wärmeleistung als Wellenleistung abführt und die an der Gasturbine zugeführte Wärmeleistung ca. ein Drittel der der Gasturbine und dem Reaktor insgesamt zugeführten Wärmeleistung ausmacht, wird ein Neuntel der gesamten Wärmeleistung dem Verbrennungsabgas in Form der Wellenleistung entzogen. Entsprechend verringert sich damit auch die adiabatische Verbrennungstemperatur um ca. ein Neuntel.

Die vorliegende Erfindung schlägt daher, wie erläutert, vor, nicht das Verbrennungsabgas insgesamt in den Reaktor einzuspeisen und zur Unterstützung der Verbrennung des Brennstoffs zu verwenden, sondern höchstens einen Teil hiervon. Dem Reaktor wird im Gegensatz zu herkömmlichen gekoppelten Anlagen also teilweise oder ausschließlich externes Verbrennungsunterstützungsgas, das nicht aus dem Verbrennungsabgas gebildet wird, beispielsweise frische Verbrennungsluft, zugeführt. Die eigentliche Kopplung der Gasturbine mit einem entsprechenden Reaktor erfolgt dabei mittels einer Vorwärmeinrichtung, die beispielsweise einen oder mehrere geeignete Wärmetauscher für einen indirekten Wärmetausch umfasst. Durch den indirekten Wärmetausch des Verbrennungsabgases mit dem Verbrennungsunterstützungsgas wird damit zwar dessen Temperatur genutzt (also Wärmeleistung übertragen), aber der Sauerstoffgehalt des Verbrennungsunterstützungsgases nicht beeinflusst. Auf diese Weise kann beispielsweise Frischluft mit ca. 21% Sauerstoff als Verbrennungsunterstützungsgas erwärmt und in den Reaktor eingespeist werden. Hierdurch lassen sich wieder die zuvor erläuterten adiabatischen Verbrennungstemperaturen von ca. 2.000 °C (bei teilweiser Verwendung des Verbrennungsabgases nur zur Vorwärmung und teilweiser Einspeisung in den Reaktor) und sogar mehr (bei ausschließlicher Verwendung zur Vorwärmung) erzielen, wie nachfolgend erläutert.

Die erwähnte teilweise Verwendung des Verbrennungsabgases nur zur Vorwärmung einerseits und die teilweise Einspeisung in den Reaktor andererseits umfasst beispielsweise, einen Teil des Verbrennungsabgases mit "frischem" Verbrennungsunterstützungsgas, beispielsweise Luft, zu mischen und dabei einen definierten Sauerstoffgehalt einzustellen. Beispielsweise kann dabei ein Sauerstoffgehalt von ca. 19% eingestellt werden. Ein anderer Teil des Verbrennungsabgases wird nicht in den Reaktor eingespeist, sondern nur zur Vorwärmung des Verbrennungsunterstützungsgases durch indirekten Wärmetausch verwendet. Bei einer angenommenen Temperatur des Verbrennungsabgases von ca. 600 °C kann die erwähnte adiabatische Verbrennungstemperatur von ca. 2.000 °C in dem Reaktor und damit ein vergleichbarer Strahlungszonenwirkungsgrad wie in herkömmlichen Reaktoren erzielt werden. Der Reaktor braucht daher in seiner Betriebsweise nicht oder nur geringfügig angepasst zu werden.

Ein weiterer wesentlicher Vorteil der vorliegenden Erfindung ist der, dass, selbst wenn die Gasturbine ausfällt oder einer Wartung bedarf, der Reaktor weiterhin betrieben werden kann. In diesem Fall kann beispielsweise nicht vorgewärmte Luft als Verbrennungsunterstützungsgas eingesetzt werden. Alternativ ist in diesem Fall auch eine anderweitige Vorwärmung des Verbrennungsunterstützungsgases, beispielsweise mittels Dampf und/oder Rauchgas, möglich. Entsprechende Vorwärmeinrichtungen müssen dabei nur für einen kurzzeitigen Betrieb ausgelegt werden und sind entsprechend kostengünstig.

Es sei an dieser Stelle darauf hingewiesen, dass auch in herkömmlichen Anlagen, wie sie beispielsweise in Figur 3 veranschaulicht sind, zusätzliches Verbrennungsunterstützungsgas zusätzlich zu dem vollständig in den Reaktor eingespeisten Verbrennungsabgas der Gasturbine zugespeist werden kann. Dies erfolgt jedoch herkömmlicherweise nur zur Schaffung einer Regelgröße zur Erhöhung der Unabhängigkeit zwischen Gasturbine und Reaktor. Das Problem des verringerten Sauerstoffgehalts und des verschlechterten Strahlungszonenwirkungsgrads in dem Reaktor kann hierdurch prinzipbedingt nicht gelöst werden.

Im Rahmen der vorliegenden Erfindung lässt sich demgegenüber der Strahlungszonenwirkungsgrad des Reaktors deutlich steigern. Der Reaktor kann dabei bei dem Strahlungszonenwirkungsgrad, wie er auch in einem autarken Reaktor zu erzielen ist, betrieben werden, wie soeben erläutert. Es ist jedoch auch möglich, den Strahlungszonenwirkungsgrad durch die Vorwärmung und die Erzielung noch höherer Temperaturen im Reaktor weiter zu erhöhen.

Nachfolgend werden die in den Patentansprüchen angegebenen Ausführungsformen der vorliegenden Erfindung, die bereits oben teilweise erläutert wurden, nochmals zusammengefasst:

Insbesondere eignet sich das Verfahren der vorliegenden Erfindung zum Einsatz bei den eingangs erwähnten Rohrreaktoren, d.h. bei Anlagen, in denen der wenigstens eine Reaktor als Rohrreaktor ausgebildet ist, in dem in einer Strahlungszone Reaktionsrohre von außen durch Brenner beheizt werden, in denen der Brennstoff verbrannt wird. Herkömmliche, autark betriebene Reaktoren umfassen Einspeiseöffnungen, durch die das Verbrennungsunterstützungsgas eingespeist wird. Innerhalb des Reaktors bzw. einer Brennkammer eines entsprechenden Reaktors liegt ein leichter Unterdruck vor, der durch ein Gebläse im Rauchgaskanal erzeugt wird. Ein Verbrennungsunterstützungsgas wird daher automatisch angesaugt. Im Gegensatz dazu kann die vorliegende Erfindung umfassen, ein Verbrennungsunterstützungsgas unter leichtem Überdruck mittels eines Gebläses in einen entsprechenden Reaktor bzw. dessen Brennkammer einzuspeisen. Eine solche Einspeisung ist beispielsweise für eine Luftvorwärmung bei Verfahren zur Wasserstoffreformierung typisch.

Das Verfahren der vorliegenden Erfindung eignet sich in besonderer Weise für die eingangs erwähnten Dampfspaltverfahren, d. h. für Verfahren, bei denen zur Herstellung der (olefinischen) Kohlenwasserstoffe ein Kohlenwasserstoffe enthaltender Einsatz mit Dampf durch die Reaktionsrohre des als Rohrreaktor ausgebildeten Reaktors geführt wird. In entsprechenden Dampfspaltverfahren liegen die zuvor erwähnten Temperaturen in der Strahlungszone vor. In gleicher Weise eignet sich die Erfindung jedoch auch für katalytische Verfahren, beispielsweise die eingangs erwähnten Verfahren zur Alkandehydrierung, d.h. für Verfahren, die Reaktoren umfassen, bei denen in den Reaktionsrohren ein Katalysator bereitgestellt ist, oder Verfahren zur Wasserstoffreformierung.

Wie bereits erwähnt, ist das erfindungsgemäße Verfahren insbesondere deshalb vorteilhaft, weil sich durch die hierbei erzielbaren Temperaturen ein hoher Strahlungszonenwirkungsgrad des Reaktors erreichen lässt. Dies bedeutet, mit anderen Worten, dass das Verfahren in Fällen zum Einsatz kommt, in denen zumindest ein Bereich des wenigstens einen Reaktors durch die Beheizung unter Verwendung des Brennstoffs und des Verbrennungsunterstützungsgases auf eine adiabatische Verbrennungstemperatur von typischerweise 1.500 bis 2.500 °C aufgeheizt wird.

Als Verbrennungskraftmaschinen zum Einsatz in der vorliegenden Erfindung eignen sich insbesondere Gasturbinen, weil diese bei gutem mechanischem bzw. elektrischem Wirkungsgrad eine hohe Nennleistung bei relativ geringen Kosten aufweisen. Gasturbinen werden daher auch typischerweise in Kraftwerken eingesetzt. Der mechanische Wirkungsgrad einer Gasturbine alleine ist typischerweise nicht höher als jener eines entsprechend ausgebildeten Dieselmotors oder eines Kohle-Dampfkraftwerks. Da die Temperatur des Verbrennungsabgases bei einem Dieselmotor bei ca. 600 °C und eines Benzinmotors bei ca. 700 bis 1.000 °C liegt, eignen sich derartige Verbrennungskraftmaschinen zum Einsatz in der vorliegenden Erfindung ebenfalls.

Während herkömmlicherweise ein Nachteil bei der Verwendung einer Gasturbine im eingesetzten relativ hochwertigen Brennstoff (Gas) liegt, besteht hierin gerade ein Vorteil der vorliegenden Erfindung: In den hier in Frage kommenden Verfahren zur Herstellung von Reaktionsprodukten (beispielsweise bei Dampfspaltverfahren und der Wasserstoffreformierung) wird ein aus verbrennungstechnischer Sicht hochwertiges sogenanntes Tailgas als Restgas gewonnen. Es handelt sich dabei um eine methanhaltige Fraktion bzw. ein Gemisch aus Kohlenmonoxid, Kohlendioxid und Wasserstoff (Synthesegas). Das im Rahmen der Erfindung durchgeführte (Teil-)Verfahren zur Herstellung der Kohlenwasserstoffe liefert damit einen geeigneten Brennstoff für eine Gasturbine. Selbstverständlich kann ein entsprechendes Gasgemisch auch in einem Motor verfeuert werden. Auf diese Weise wird eine weitere synergistische Integration entsprechender Anlagenteile vorgenommen.

Besonders vorteilhaft ist es, wenn das Verbrennungsabgas der Verbrennungskraftmaschine auf einem Temperaturniveau von weniger als 650 °C bereitgestellt wird, da sich in diesem Fall ein Verbrennungsunterstützungsgas besonders effektiv und kostengünstig erwärmen lässt. Die Materialkosten, beispielsweise für eingesetzte Wärmetauscher, sind bei entsprechenden Temperaturen noch ausgesprochen gering. Allgemein kann das Verbrennungsabgas der Verbrennungskraftmaschine jedoch auf einem Temperaturniveau von 500 bis 1.000 °C, insbesondere auf einem Temperaturniveau von 600 bis 700 °C oder auf einem Temperaturniveau von 500 bis 650 °C, bereitgestellt werden.

In einem Verfahren gemäß einer Ausführungsform der Erfindung wird vorteilhafterweise ein Teil des Verbrennungsabgases der Verbrennungskraftmaschine zur Erwärmung des Verbrennungsunterstützungsgases durch indirekten Wärmetausch verwendet und ein Teil des Verbrennungsabgases der Verbrennungskraftmaschine mit dem Verbrennungsunterstützungsgas vereinigt und zusammen mit diesem den wenigstens einen Reaktor zugeführt. Diese teilweise Verwendung des Verbrennungsabgases zur Vorwärmung und teilweise zur Einspeisung in den Reaktor ermöglicht eine besonders günstige Kombination einer Gasturbine bzw. einer anderen Verbrennungskraftmaschine und einem Reaktor. In diesem Fall können die Bedingungen in dem Reaktor an jene herkömmlicher, autarker Reaktoren approximiert werden, so dass keine oder nur geringe Änderungen in der Betriebsweise entsprechender Reaktoren und/oder ihrer konstruktiven Ausgestaltung erforderlich sind. Die Reaktionsrohre und die Einrichtungen zur Abwärmenutzung (in der sogenannten Konvektionszone) können beibehalten werden.

In neuen Anlagen kann es sich jedoch auch als vorteilhaft erweisen, das Verbrennungsabgas der Verbrennungskraftmaschine vollständig zur Erwärmung des Verbrennungsunterstützungsgases durch indirekten Wärmetausch zu verwenden und nicht dem wenigstens einen Reaktor zuzuführen. Der wenigstens eine Reaktor erhält daher den vollständigen Sauerstoffgehalt des Verbrennungsunterstützungsgases, beispielsweise von Luft, zusätzlich zur Wärme des Verbrennungsabgases, so dass sich die Temperaturen in einem entsprechenden Reaktor weiter steigern lassen. Auf diese Weise erhöht sich der Strahlungszonenwirkungsgrad eines entsprechenden Reaktors nochmals beträchtlich. Der Brennstoffverbrauch am Reaktor lässt sich hierdurch entsprechend verringern.

Die vorliegende Erfindung eignet sich insbesondere zum Einsatz mit Erdgas, einem methanhaltigen Gasgemisch und/oder Synthesegas als Brennstoff und/oder Luft als Verbrennungsunterstützungsgas. Entsprechende Brennstoffe können, wie erwähnt, auch typische Restgase entsprechender Verfahren zur Herstellung von Reaktionsprodukten (beispielsweise aus Verfahren zur Dampfspaltung, Sythesegaserzeugung oder Wasserstoffreformierung) sein. Die vorliegende Erfindung ermöglicht es insbesondere, durch höhere Effizienz Brennstoff einzusparen.

Ein weiterer Vorteil des erfindungsgemäßen Verfahrens ergibt sich, wenn aus Abwärme des wenigstens einen Reaktors Druckdampf erzeugt und zum Antrieb wenigstens einer Welle, insbesondere einer Welle eines Generators, verwendet wird. Auf diese Weise kann weitere mechanische Leistung gewonnen und sinnvoll genutzt werden, selbst wenn entsprechender Druckdampf auf geringerem Druck vorliegt. Bei dem mittels der Abwärme des wenigstens einen Reaktors gewonnenen Dampf handelt es sich im Grunde lediglich um ein Nebenprodukt, mittels dessen man die nicht für die Reaktion nutzbare Wärme (Abwärme) einer sinnvollen Verwendung zuführt. Im theoretischen Idealfall würde im Reaktor lediglich Reaktionswärme und keine Abwärme, also kein Dampf, erzeugt.

Der Vorteil der vorliegenden Erfindung ist der, dass die Menge des mittels der Abwärme des oder der Reaktoren erzeugte Druckdampf minimiert werden kann. Der mittels der Abwärme des oder der Reaktoren erzeugte Druckdampf wird mit deutlich höheren Exergieverlusten gewonnen als im (mehrstufigen und in der Spitze bei höheren Drücken/Temperaturen effizienteren) Kraftwerksdampfprozess. Druckdampf kann beispielsweise in Dampfspaltverfahren teilweise als Heizdampf zur Vorwärmung des oder der Einsatzströme mit nahezu 100% Wirkungsgrad eingesetzt werden. Im Fall der Erzeugung von mechanischer Leistung in einer Turbine ist der Wirkungsgrad um ca. den Faktor zwei schlechter als in einem Dampfkraftwerksverfahren.

Zu den Merkmalen und Vorteilen der erfindungsgemäß vorgesehenen Anlage zur Erzeugung von mechanischer Leistung und zur Herstellung von Kohlenwasserstoffen, die insbesondere zur Durchführung eines Verfahrens eingerichtet ist, wie es zuvor erläutert wurde, sei auf die obigen Ausführungen ausdrücklich verwiesen.

Die Erfindung sowie spezifische Ausführungsformen der Erfindung werden in den beigefügten Zeichnungen im Vergleich zum Stand der Technik erläutert.

Kurze Beschreibung der Zeichnungen

Figur 1 zeigt ein Gas-und-Dampf-Kraftwerk gemäß dem Stand der Technik in vereinfachter schematischer Darstellung.

Figur 2 zeigt einen gemäß dem Stand der Technik betriebenen befeuerten Reaktor in vereinfachter schematischer Darstellung.

Figur 3 zeigt eine Anlage mit einer Gasturbine und einem befeuerten Reaktor gemäß dem Stand der Technik in vereinfachter schematischer Darstellung.

Figur 4 zeigt eine Anlage mit einer Gasturbine und einem befeuerten Reaktor gemäß einer Ausführungsform der Erfindung in vereinfachter schematischer Darstellung.

Figur 5 zeigt eine Anlage mit einer Gasturbine und einem befeuerten Reaktor gemäß einer Ausführungsform der Erfindung in vereinfachter schematischer Darstellung.

In den Figuren sind einander entsprechende Elemente mit identischen Bezugszeichen angegeben und werden der Übersichtlichkeit halber nicht wiederholt erläutert. Entsprechendes gilt auch für die jeweils gezeigten Fluidströme, die mit Kleinbuchstaben angegeben sind, selbst wenn diese in unterschiedlichen Mengenströmen bereitgestellt werden, wie nachfolgend erläutert.

In allen dargestellten Beispielen ist ein Reaktor, falls gezeigt, zur Durchführung eines Dampfspaltverfahrens eingerichtet, wird also mit einem kohlenwasserstoffhaltigen Einsatzstrom beschickt, der mit Dampf vermischt ist. Als Brennstoff wird ein geeignetes Brenngas wie oben erläutert, als Verbrennungsunterstützungsgas Luft eingesetzt. Die gezeigten Einrichtungen eignen sich jedoch grundsätzlich auch zur Durchführung anderer Verfahren zur Herstellung von Reaktionsprodukten bzw. Verwendung anderer Brennstoffe und Verbrennungsunterstützungsgase. Wenngleich nachfolgend häufig von "einem" Reaktor bzw. "einer" Gasturbine die Rede ist, können entsprechende Anlagen auch mehrere Reaktoren bzw. Gasturbinen umfassen.

Ausführliche Beschreibung der Zeichnungen

In Figur 1 ist ein Gas-und-Dampf-Kraftwerk gemäß dem Stand der Technik vereinfacht schematisch dargestellt und insgesamt mit 300 bezeichnet.

Das Gas-und-Dampf-Kraftwerk 300 umfasst als zentrale Komponenten eine Gasturbine 1, die, wie eingangs erläutert, eine Verdichtungsstufe 11 und eine Expansionsstufe 12 sowie eine zwischen der Verdichtungsstufe 11 und der Expansionsstufe 12 angeordnete, hier jedoch nicht gesondert dargestellte Brennkammer aufweist. Mittels der Gasturbine 1 wird ein Generator G angetrieben. Der Gasturbine 1 wird ein Verbrennungsunterstützungsgas a zugeführt und in der Verdichtungsstufe 11 verdichtet. In die nicht dargestellte Brennkammer der Gasturbine 1 wird ein Brennstoff b eingespeist und in der Brennkammer in einer durch das Verbrennungsunterstützungsgas a geschaffenen Atmosphäre unter Druck verbrannt.

Typischerweise erfolgt die Verbrennung dabei bei deutlich überstöchiometrischem Sauerstoffangebot, beispielsweise bei einem Lambdawert von ca. 3, so dass ein bei der Verbrennung gebildetes Verbrennungsabgas c (Heißgas), das in der Expansionsstufe 12 der Gasturbine entspannt wird, noch einen beträchtlichen Sauerstoffgehalt aufweist. Wird als Verbrennungsunterstützungsgas a Luft mit einem natürlichen Sauerstoffgehalt von ca. 21% verwendet, enthält das Verbrennungsabgas c beispielsweise noch einen Sauerstoffgehalt von ca. 14%.

Das Verbrennungsabgas c, das beispielsweise bei einer Temperatur von 600 °C vorliegen kann, wird in dem Gas-und-Dampf-Kraftwerk 300 einem Abhitzedampferzeuger 5 zugeführt. Dem Abhitzedampferzeuger 5 wird typischerweise allenfalls wenig weiterer Brennstoff zugeführt, d.h. in dem Abhitzedampferzeuger 5 wird hauptsächlich die fühlbare Wärme des Verbrennungsabgases c genutzt. Den Abhitzedampferzeuger 5 verlässt ein entsprechend abgekühltes Verbrennungsabgas g.

In der stark vereinfachten Darstellung der Figur 1 wird dabei Druckdampf f erzeugt. Typischerweise wird in entsprechenden Gas-und-Dampf-Kraftwerken 300 Druckdampf f auf drei Druckniveaus erzeugt. Die Druckniveaus betragen z.B. ca. 130, 30 und 8 bar, wobei Dampf auf mittlerem und niedrigem Druckniveau zum Teil einer Turbine auf einem Zwischendruck entnommen wird (durch "Anzapfen") und Dampf auf mittlerem Druckniveau ausgehend von der Sattdampftemperatur auf bis etwa 570 °C erhitzt wird ("Zwischenüberhitzung"). Dieser Aufwand dient dazu, Exergieverluste dadurch zu minimieren, dass der Wärmeübergang vom Verbrennungsabgas c auf Speisewasser bzw. Dampf mit möglichst geringem Temperaturunterschied erfolgt.

Der Druckdampf f wird in einer Entspannungsturbine 6 (Dampfturbine) zur Erzeugung von Wellenleistung (mechanischer Leistung) genutzt. Diese Leistung wiederum wird mittels eines Generators G in elektrische Leistung umgewandelt. Dieser Generator kann derselbe wie der mit der Gasturbine 1 gekoppelte Generator G sein oder wird separat bereitgestellt. Der entspannte Dampfstrom (in Figur 1 ohne Bezeichnung) wird in einem Kühler 7, beispielsweise mittels Kühlwasser, abgekühlt. Das anfallende Dampfkondensat wird dem Prozess wieder zugeführt (über eine sogenannte Kesselspeisewasserpumpe).

Nachfolgend sind typische Kenngrößen eines Gas-und-Dampf-Kraftwerks 300 veranschaulicht. Die entsprechenden Variablen sind für eine elektrische Nettoleistung von 100 MW angegeben, da dies die Größenordnung ist, wie sie bei einer dem technischen Stand entsprechenden großen Anlage zum Dampfspalten benötigt würde. Im Kraftwerksbereich sind Nettoleistungen von 80 bis 400 MW pro Gasturbineneinheit typisch. Es werden dabei ca. 619.000 Normkubikmeter pro Stunde ($Nm^3/h$) Verbrennungsluft als Verbrennungsunterstützungsgas a sowie eine Unterfeuerungsleistung in Form des Brennstoffs b von ca. 180 MW eingesetzt.

Entsprechende Werte sind unten nochmals tabellarisch zusammengefasst. Rundungsfehler wurden vernachlässigt.

Im erläuterten Fall werden typischerweise ca. 640.000 $Nm^3/h$ des Verbrennungsabgases c gebildet. Auch die Menge des abgekühlten Verbrennungsabgases g beträgt in diesem Fall ca. 640.000 $Nm^3/h$, wenn keine Zusatzfeuerung im Abwärmedampferzeuger 5 erfolgt.

Typischerweise liegt der elektrische Wirkungsgrad der Gasturbine 1 und des damit verbundenen Generators bei ca. 0,36 (36%). Der elektrische Wirkungsgrad der Entspannungsturbine 6 liegt, bezogen auf die dem Abhitzedampferzeuger 5 zugeführte Energie bei ca. 0,32 bzw. bezogen auf die gesamte eingesetzte Energie bei ca. 0,20. Der Anteil der Entspannungsturbine 6 an der gesamten elektrischen Leistung eines entsprechenden Gas-und-Dampf-Kraftwerks liegt im dargestellten Beispiel beispielsweise ebenfalls bei ca. 0,36. Der thermische Wirkungsgrad ohne Berücksichtigung des Kühlers 7 beträgt im Beispiel ca. 0,82. (Der thermische Wirkungsgrad hängt von Kondensationstemperatur, Dampfmenge usw. ab und bewegt sich im Bereich zwischen ca. 0,75 und ca. 0,85.) Der thermische Wirkungsgrad ist im vorliegenden Fall häufig wenig aussagekräftig, da, selbst wenn die überwiegende Wärme dem Rauchgas entzogen wird, beispielsweise die bloße Bereitstellung von Heißwasser oder Dampf ohne dessen Nutzbarkeit mit hohem Wirkungsgrad die Effizienz entsprechender Anlagen kaum steigert.

Von den ca. 180 MW Unterfeuerungsleistung des Brennstoffs b werden vor diesem Hintergrund in dem mit der Gasturbine 1 gekoppelten Generator G ca. 64 MW als elektrische Leistung gewonnen, ca. 112 MW gehen als fühlbare Wärme in das Verbrennungsabgas c über, Wärmeverluste entsprechen typischerweise ca. 3 MW. Wiederum verbleiben von den ca. 112 MW fühlbarer Wärme des Verbrennungsabgases c in dem abgekühlten Verbrennungsabgas g typischerweise ca. 33 MW, wobei das abgekühlte Verbrennungsabgas g dabei auf eine Temperatur von ca. 128 °C abgekühlt wird. Von den verbleibenden ca. 80 MW werden 36 MW als elektrische Leistung in dem mit der Entspannungsturbine 6 gekoppelten Generator G gewonnen und ca. 44 MW in dem Kühler 7 abgeführt.

Bei einer gesamten Heizleistung von ca. 180 MW und einer elektrischen Leistung von ca. 100 MW der beiden Generatoren G liegt damit der elektrische Gesamtwirkungsgrad eines Gas-und-Dampf-Kraftwerks 300 im Beispiel bei ca. 0,56. Im Testlauf wurden bei großen Gas-und-Dampf-Kraftwerken (mit ca. 800 MW Leistung) Wirkungsgrade von maximal 0,61 erzielt, die aber bei wärmerem Kühlwasser deutlich sinken.

In Figur 2 ist ein befeuerter Reaktor gemäß dem Stand der Technik vereinfacht schematisch dargestellt und insgesamt mit 2 bezeichnet. Wie eingangs erläutert, können entsprechende befeuerte Reaktoren typischerweise zur Erzeugung von Kohlenwasserstoffprodukten oder Synthesegas, beispielsweise durch Dampfspalten, eingesetzt werden. Ein entsprechender Reaktor 2 umfasst, wie allgemein bekannt, typischerweise eine Strahlungszone 21 und eine Konvektionszone 22. In der Strahlungszone 21 sind typischerweise mehrere Brenner angeordnet (nicht dargestellt), denen ein Brennstoff d zugeführt wird. Die Verbrennung wird durch die Zuführung eines Verbrennungsunterstützungsgases e ermöglicht. In der Strahlungszone 21 und der Konvektionszone 22 sind typischerweise Reaktionsrohre angebracht, die mit entsprechenden Brennern von außen beheizt werden.

Auch in einem befeuerten Reaktor 2 wird Abwärme zum Großteil zur Erzeugung von Druckdampf f genutzt, dieser ist jedoch typischerweise vergleichsweise schlecht geeignet, um mit zufriedenstellendem Wirkungsgrad zur Gewinnung von elektrischer Energie eingesetzt zu werden. Die schlechtere Verwertbarkeit des Druckdampfs f aus einem typischen befeuerten Reaktor 2, wie er in Figur 2 dargestellt ist, ergibt sich aus seiner vergleichsweise niedrigen Temperatur und seinem vergleichsweise niedrigen Druck sowie der Tatsache, dass nur ein Dampfniveau realisiert wird (und daher dem vergleichsweise großem Exergieverlust bei der Dampferzeugung). Während in typischen Gas-und-Dampf-Kraftwerken, beispielsweise einem Gas-und-Dampf-Kraftwerk 300, wie es in Figur 1 dargestellt ist, Druckdampf f mit 130 bar und 570 °C gewonnen wird, beträgt der Druck des Druckdampfs f aus dem befeuerten Reaktor 2, wie er in Figur 2 dargestellt ist, typischerweise nur 120 bar, seine Temperatur typischerweise nur 520 °C. Typischerweise wird entsprechender Druckdampf f aus befeuerten Reaktoren 2 zur Gewinnung von Wellenleistung (z.B. in einer Anlage zum Dampfspalten) und als Heizdampf eingesetzt. Auch hier wird ein abgekühltes Verbrennungsabgas g erhalten.

Nimmt man bei einer entsprechenden bilanzierenden Betrachtung eine Unterfeuerungsleistung von ca. 1.000 MW (verteilt auf typischerweise mehrere Reaktoren) in Form des Brennstoffs d an und geht man von einer Zufuhr von beispielsweise ca. 1.067.000 $Nm^3$/h Verbrennungsluft als dem Verbrennungsunterstützungsgas e aus, können bei einem typischen Strahlungszonenwirkungsgrad von ca. 0,42 (es handelt sich hierbei um einen typischen Wert für einen Reaktor für ein Dampfspaltverfahren) in der Strahlungszone 21 aus der Abwärme des Reaktors 2 ca. 512 MW bzw. ca. 595 t/h Druckdampf f gewonnen werden. Es gehen ca. 60 MW in das abgekühlte Rauchgas g über, das in einer Menge von ca. 1.172.000 $Nm^3$/h und bei einer Temperatur von ca. 128 °C entnommen wird. Die "fehlende" Wärmeleistung von 428 MW wird in Form chemischer Bindungsenergie und fühlbarer Wärme im rohrseitigen Prozessgas, also nicht im Rauchgasstrom sondern aus der Reaktionszone des Reaktors 2, abgeführt. Dieser Wert ist für alle hier beispielhaft veranschaulichten Reaktoren 2 der nachfolgenden Figuren gleich, da die gleiche Menge Reaktionsprodukt erzeugt wird.

In Figur 3 ist eine kombinierte Anlage mit einer Gasturbine 1 und einem befeuerten Reaktor 2 gemäß dem Stand der Technik vereinfacht schematisch dargestellt und insgesamt mit 400 bezeichnet. Die Grundidee bei der Bereitstellung einer derartigen Anlage 400 ist die, fühlbare Wärme eines Verbrennungsabgases c einer Gasturbine 1 ähnlich wie in einem Gas-und-Dampf-Kraftwerk, beispielsweise einem Gas-und-Dampf-Kraftwerk 300, wie es in Figur 1 dargestellt ist, in einem entsprechenden befeuerten Reaktor 2 zu nutzen. Hierbei macht man sich die erwähnte Tatsache zu Nutze, dass das Verbrennungsabgas c aufgrund der deutlich überstöchiometrischen Verbrennung in der Gasturbine 1 noch einen beträchtlichen Sauerstoffgehalt aufweist. Zusätzliches Verbrennungsunterstützungsgas d, beispielsweise Luft, wird im dargestellten Beispiel dennoch zugeführt, beispielsweise mittels eines Gebläses 3 dem Verbrennungsabgas c zugespeist. Diese zusätzliche Zufuhr dient zur Schaffung einer zusätzlichen Regelgröße zur Regelung der Verbrennung in dem Reaktor 2.

Ein beträchtlicher Nachteil an derartigen kombinierten Anlagen 400 gemäß dem Stand der Technik ist jedoch der, dass es zu einer deutlichen Verringerung des Strahlungszonenwirkungsgrads des befeuerten Reaktors 2 in der Strahlungszone 21 kommt. Im Vergleich zu einem autarken Reaktor 2, wie er beispielsweise in Figur 2 dargestellt ist, verringert sich der Strahlungszonenwirkungsgrad beispielsweise von ca. 0,42 auf ca. 0,37. Diese Tatsache ist insbesondere darauf zurückzuführen, dass das Verbrennungsabgas c zwar eine vergleichsweise hohe Temperatur von beispielsweise ca. 600 °C aufweist, sein Sauerstoffgehalt mit beispielsweise ca. 14% jedoch deutlich unterhalb jenes typischerweise verwendeter Verbrennungsunterstützungsgase wie Verbrennungsluft liegt. Dies kann auch nicht durch die Zufuhr weiterer Verbrennungsluft d bzw. eines entsprechenden Verbrennungsunterstützungsgases ausgeglichen werden (zumindest nicht ohne eine außerordentlich aufwendige Sauerstoffanreicherung). Wird in einem autark betriebenen Reaktor 2, wie er in Figur 2 veranschaulicht ist, Luft mit ca. 21% Sauerstoff als Verbrennungsunterstützungsgas d verwendet, lassen sich durch die Verbrennung in der Strahlungszone 21 des Reaktors 2 noch adiabatische Verbrennungstemperaturen von ca. 2.000 °C erreichen. Im Gegensatz dazu ist in einer Anlage 400, wie sie in Figur 3 gezeigt ist, die adiabatische Verbrennungstemperatur in der Strahlungszone 21 aufgrund der zuvor erläuterten Sachverhalte auf ca. 1.750 °C beschränkt. Dies schlägt sich direkt in dem angegebenen verschlechterten Strahlungszonenwirkungsgrad nieder.

In bilanzmäßiger Betrachtung wird ein beträchtlicher Anteil der in dem Verbrennungsunterstützungsgas a enthaltenen chemischen Energie bereits in der Gasturbine 1 entzogen und steht daher anschließend nicht mehr in dem Verbrennungsabgas c zur Verfügung.

Nachfolgend werden Beispieldaten für eine Gasturbine stromauf eines oder mehrerer mit einer Leistung von 1.000 MW betriebener Reaktoren zum Dampfspalten mit maximierter Gasturbinenleistung, d.h. minimalem Einsatz von zusätzlichem Verbrennungsunterstützungsgas d zur Regelung, angegeben. Setzt man in einer solchen Anlage 400 beispielsweise ca. 1.132.000 Nm$^3$/h Verbrennungsluft als Verbrennungsunterstützungsgas a ein und verwendet eine Unterfeuerungsleistung von ca. 348 MW in Form des Brennstoffs b, so lassen sich bei den zuvor erläuterten Wirkungsgraden in dem mit der Gasturbine 1 gekoppelten Generator G ca. 118 MW elektrischer Leistung gewinnen. Es gehen ca. 224 MW als fühlbare Wärme in das Verbrennungsabgas c über. Es ergeben sich Verluste von ca. 5 MW, insbesondere am Generator G und Nebenaggregaten sowie am Ölkühler der Gasturbine 1. Das Verbrennungsabgas c wird in einer Menge von ca. 1.170.000 Nm$^3$/h gebildet.

Im dargestellten Beispiel werden als Verbrennungsunterstützungsgas d beispielsweise ca. 189.000 Nm$^3$/h Verbrennungsluft verwendet. Die Unterfeuerungsleistung in Form des Brennstoffs e beträgt ca. 922 MW. Die gesamte eingesetzte Heizleistung in Form der Brennstoffe b und e beträgt damit ca. 1.270 MW, die in der Strahlungszone 21 verfügbare Heizleistung aus der fühlbaren Wärme des Verbrennungsabgases c und der Unterfeuerungsleistung in Form des Brennstoffs e ca. 1.147 MW. Von diesen werden ca. 650 MW in Form des Druckdampfs f in einer Menge von ca. 756 t/h zurückgewonnen, ca. 69 MW gehen in das abgekühlte Rauchgas g über, das, wie zuvor, bei ca. 128 °C entnommen wird. Die Menge des abgekühlten Rauchgases g beträgt damit ca. 1.457.000 Nm$^3$/h gegenüber den erwähnten ca. 1.172.000 Nm$^3$/h in einem autarken Reaktor 2, wie er in Figur 2 dargestellt ist. Auch hier werden 428 MW als chemische Bindungsenergie und fühlbare Wärme im rohrseitigen Prozessgas abgeführt, da hier die gleiche Menge Reaktionsprodukt wie in dem Reaktor 2 gemäß Figur 2 erzeugt werden soll.

Wie erwähnt, verringert sich der Strahlungszonenwirkungsgrad in der Strahlungszone 21 auf ca. 0,37. Der Wirkungsgrad der Dampferzeugung (aus Druckdampf f) beträgt ca. 0,51, der gesamte thermische Wirkungsgrad ca. 0,94. (Ein Teil der Wärme, die in der Strahlungszone 21 auf das Prozessgas übergeht, wird zur Dampferzeugung genutzt. Die beiden Wirkungsgrade dürfen daher nicht addiert werden bzw. müssen sich nicht zu dem genannten thermischen Wirkungsgrad ergänzen. Zur vollständigen Bilanzierung fehlt die Wärmemenge und chemische Bindungsenergie, die mit dem Prozessgas abgeführt wird. Auch diese Werte sind jedoch in allen in den vorliegenden Figuren veranschaulichten Reaktoren 2 gleich.)

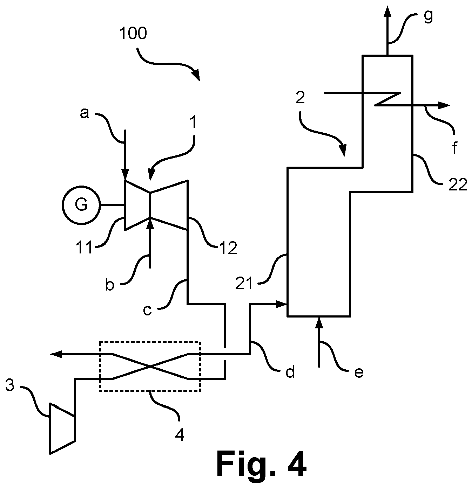

In Figur 4 ist eine kombinierte Anlage mit einer Gasturbine 1 und einem befeuerten Reaktor 2 gemäß einer Ausführungsform der Erfindung vereinfacht schematisch dargestellt und insgesamt mit 100 bezeichnet.

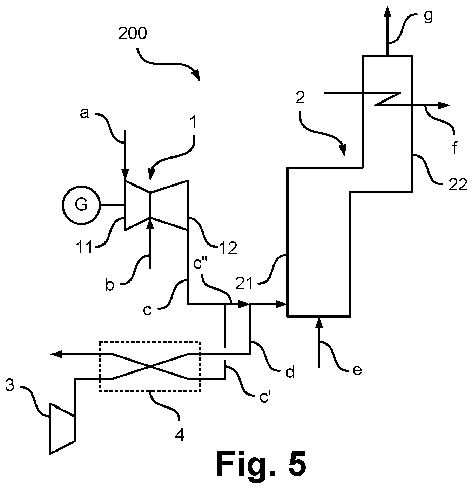

Ein zentraler Aspekt der vorliegenden Erfindung ist, wie zuvor mehrfach erläutert, die Verwendung einer Vorwärmeinheit 4, mittels welcher in den Reaktor 2 eingespeistes Verbrennungsunterstützungsgas b vorgewärmt wird. In dem in der Figur 4 dargestellten Beispiel wird das gesamte Verbrennungsabgas c aus der Gasturbine 1 durch die Vorwärmeinheit 4 geführt, es kann jedoch auch vorgesehen sein, nur einen Teil des Verbrennungsabgases c entsprechend zu verwenden. Letzteres ist in der nachfolgenden Figur 5 gezeigt. Mittels der Vorwärmeinheit 4, die beispielsweise einen oder mehrere geeignet ausgebildeter Wärmeaustauscher umfassen kann, kann fühlbare Wärme des Verbrennungsabgases 4 auf das Verbrennungsunterstützungsgas d übertragen werden.

Hierdurch ergibt sich der besondere Vorteil, dass in den Reaktor 2 ein Verbrennungsunterstützungsgas d, beispielsweise Verbrennungsluft, mit weiterhin hohem Sauerstoffgehalt eingespeist werden kann, das jedoch gleichzeitig mit der fühlbaren Wärme des Verbrennungsabgases c erwärmt werden kann. Wie sich überraschend herausgestellt hat, lässt sich hierdurch der Strahlungszonenwirkungsgrad in der Strahlungszone 21 des Reaktors 2 nicht nur gegenüber Reaktoren in entsprechenden gekoppelten Anlagen 400, wie sie in Figur 3 dargestellt sind, sondern auch gegenüber autarken Reaktoren 2, wie sie in Figur 2 dargestellt sind, beträchtlich erhöhen. Als Faustregel ergibt sich bei 10 °C Vorwärmung eine Erhöhung des Strahlungszonenwirkungsgrads um 0,2%.

In der in Figur 4 gezeigten Anlage 100 ergibt sich in der Strahlungszone ein Strahlungszonenwirkungsgrad von ca. 0,47. Bei Verwendung von ca. 383.000 Nm$^3$/h Verbrennungsluft als Verbrennungsunterstützungsgas a und einer Unterfeuerungsleistung von ca. 118 MW in Form des Stroms b kann eine elektrische Leistung von ca. 40 MW mit der Gasturbine 1 bzw. dem entsprechenden Generator G gewonnen werden. Das Verbrennungsabgas c liegt hierbei bei ca. 656 °C (hierbei handelt es sich um einen Beispielwert, typisch sind 550 bis 700 °C), entsprechend einer fühlbaren Wärme von ca. 76 MW, vor. Stromab der Vorwärmeinheit 4 beträgt die Temperatur des Verbrennungsabgases c dann noch ca. 105 °C, entsprechend einer fühlbaren Wärme von ca. 10 MW.

Die Temperatur des abgekühlten Verbrennungsabgases (Rauchgastemperatur) wird typischerweise durch den sogenannten "Schwefeltaupunkt" bestimmt. Bei dieser Temperatur kondensiert wässrige schweflige Säure, die starke Korrosion verursacht. Der Schwefeltaupunkt ist bei einem Lambdawert von 3 (wie im Rauchgas einer Gasturbine) deutlich niedriger als bei einem Lambdawert von 1,1 (bei einem Reaktor zum Dampfspalten), da anteilig eine geringere Menge (typischerweise schwefelhaltigen) Brennstoffs bzw. Verbrennungsprodukts enthalten ist. Die Beispielwerte sind für ein typisches Heizgas 105 °C einerseits bzw. 128 °C andererseits.

Die Menge des Verbrennungsabgases c beträgt ca. 395.000 Nm$^3$/h. Werden zusätzlich hierzu ca. 879.000 Nm$^3$/h Verbrennungsluft als Verbrennungsunterstützungsgas in Form des Stroms d mit beispielsweise ca. 28 °C bereitgestellt und diese in der Vorwärmeinheit 4 auf ca. 286 °C, entsprechend einer fühlbaren Wärme von ca. 66 MW, erwärmt, und beträgt die Unterfeuerungsleistung in Form des Brennstoffs e ca. 824 MW, stehen als gesamte Heizleistung ca.

942 MW und als Heizleistung in dem Reaktor 2 ca. 890 MW zur Verfügung. Von diesen ca. 890 MW ergibt sich bei dem Strahlungszonenwirkungsgrad von ca. 0,47 ein Rest von ca. 462 MW, von welchen ca. 408 MW in Form des Druckdampfs f mit ca. 475 t/h und ca. 54 MW in Form des abgekühlten Rauchgases g mit ca. 128 °C bzw. ca. 966.000 Nm$^3$/h erhalten werden.

In einer entsprechenden Anlage 100 kann das Verbrennungsunterstützungsgas d auch auf deutlich höhere Temperaturen vorgewärmt werden, wie jedoch nur tabellarisch veranschaulicht (siehe unten).

In Figur 5 ist eine kombinierte Anlage mit einer Gasturbine 1 und einem befeuerten Reaktor 2 gemäß einer weiteren Ausführungsform der Erfindung vereinfacht schematisch dargestellt und insgesamt mit 200 bezeichnet. Die Anlage 200 unterscheidet sich von der in Figur 4 gezeigten Anlage 100 dadurch, dass nur ein Teilstrom des Verbrennungsabgases c durch die Vorwärmeinheit 4 geführt wird. Dieser Teilstrom ist in der Anlage 200 mit c' bezeichnet. Ein weiterer Teilstrom, hier mit c'' bezeichnet, wird mit dem Verbrennungsunterstützungsgas d vereinigt. Auf diese Weise ergibt sich ein besonders flexibler Betrieb der Anlage 200 bzw. können die Betriebsbedingungen des Reaktors 2, wie erläutert, jenen eines autarken Reaktors 2, wie er in Figur 2 gezeigt ist, approximiert werden.

Eine entsprechende Ausführungsform der Erfindung gemäß Figur 5 bzw. Anlage 200 kann insbesondere umfassen, die Teilströme c' und c'' in einstellbaren Mengen bereitzustellen, so dass eine Anpassung an das jeweilige Wärmeangebot in dem Verbrennungsabgas c und/oder einen Wärmebedarf in dem Reaktor 2 möglich ist. Erneut seien nachfolgend beispielhafte Kenngrößen für eine Anlage 200 angegeben.

Stellt man in der Anlage 200 Verbrennungsluft als Verbrennungsunterstützungsgas a in einer Menge von ca. 1.035.000 Nm$^3$/h bereit und setzt eine Unterfeuerungsleistung in Form des Brennstoffs b von ca. 318 MW ein, lässt sich bei einem Wirkungsgrad von ca. 0,34 in der Gasturbine 1 eine elektrische Leistung von ca. 107,8 MW generieren. Der elektrische Wirkungsgrad ist in entsprechenden Anlagen etwas geringer als für Gasturbinen in einem reinen Kraftwerk (vgl. Erläuterungen zu Figur 1: Wirkungsgrad dort 0,36), da zusätzlich ein Druckverlust über den Reaktor 2 zu überwinden ist.

In dem Verbrennungsabgas c verbleibt insgesamt eine fühlbare Wärme entsprechend ca. 211 MW. Wird ein Teilstrom c' entsprechend einer Wärmemenge von ca. 77 MW bereitgestellt, kann mit diesem Teilstrom c' in der Vorwärmeinheit 4 eine fühlbare Wärme entsprechend ca. 67 MW auf Verbrennungsluft, die hier als Verbrennungsunterstützungsgas d verwendet wird, übertragen werden. Stromab der Vorwärmeinheit 4 verbleiben in dem Strom c', der in einer Menge von ca. 391.000 Nm$^3$/h pro Stunde bereitgestellt wird, ca. 10 MW an fühlbarer Wärme, was eine Temperaturverringerung von ca. 656 °C auf 105 °C (siehe Erläuterungen zu Figur 4 bezüglich Schwefeltaupunkt) entspricht.

Wie erwähnt, wird als Verbrennungsunterstützungsgas d hier beispielsweise Verbrennungsluft mittels des Gebläses 3 bereitgestellt, die auf einer (beispielhaften Umgebungs-)Temperatur von ca. 28 °C vorliegt. Die Menge der Verbrennungsluft beträgt beispielsweise ca. 397.000 Nm$^3$/h. In der Vorwärmeinheit 4 wird die Verbrennungsluft auf ca. 627 °C, entsprechend den ca. 67 MW aus dem Strom c', erwärmt. Der Teilstrom c'' des Verbrennungsabgases c wird in einer Menge von ca. 679.000 Nm$^3$/h bereitgestellt, was einer fühlbaren Wärme von ca. 134 MW entspricht. Dem Reaktor 2 wird ferner ein Brennstoff e entsprechend ca. 799 MW zugeführt.

Insgesamt stehen daher in dem Reaktor 2 eine Heizleistung von ca. 1.000 MW und in der Anlage 200 insgesamt eine Heizleistung von ca. 1.118 MW zur Verfügung. Durch eine entsprechende Einstellung der Ströme c' und c'' kann in der Strahlungszone 21 des Reaktors 2 ein Strahlungszonenwirkungsgrad von ca. 0,42 erzielt werden, der exakt jenem eines autarken Reaktors 2, wie er in Figur 2 gezeigt ist, entspricht. Es verbleiben 512 MW in dem Druckdampf f, der mit ca. 595 t/h bereitgestellt wird, und ca. 60 MW in dem abgekühlten Verbrennungsabgas c, von dem ca. 1.172.000 Nm$^3$/h bei 128 °C bereitgestellt werden.

In den nachfolgenden Tabellen 1 bis 5 sind die bereits erwähnten Mengenströme und Energiegehalte bezüglich der Figuren 1 bis 5 nochmals veranschaulicht, wobei für die Figur 4 bzw. die dort veranschaulichte Anlage 100 in den Tabellen 4A und 4B zwei Betriebsfälle, nämlich eine Vorwärmung des Verbrennungsunterstützungsgases d auf 286 °C (wobei eine herkömmliche Verfahrensführung im Reaktor 2 erfolgen kann; siehe oben) und auf 498 °C (wobei weitere prozesstechnische Änderungen am Reaktor 2 wie eine Dampferzeugung mit nur teilweiser Überhitzung oder eine externe/indirekte Einsatzvorwärmung vorzunehmen sind) dargestellt sind. In allen Fällen wird als Verbrennungsunterstützungsgas a bzw. d Luft und als Brennstoff (Rest-)Gas verwendet. Die jeweils angegebenen Werte verstehen sich als ca.-Werte unter Vernachlässigung von Rundungsfehlern. Die Wärmeleistung des Verbrennungsabgases c und des abgekühlten Verbrennungsabgases g entspricht der fühlbaren Wärme, die Wärmeleistung des Druckdampfs f der Summe aus fühlbarer Wärme und Verdampfungsenthalpie.

| Tabelle 1: Gas-und Dampf-Kraftwerk 300 (Figur 1) | |
|---|---|
| Stromproduktion Gasturbine 1 | 64 MW |
| Mengenstrom Verbrennungsunterstützungsgas a | 619.000 Nm$^3$/h |
| Feuerungsleistung Brennstoff b | 180 MW |

(fortgesetzt)

| Tabelle 1: Gas-und Dampf-Kraftwerk 300 (Figur 1) | |
|---|---|
| Wärmeverlust | 3 MW |
| Wärmeleistung Verbrennungsabgas c | 112 MW |
| Mengenstrom Verbrennungsabgas c | 640.000 Nm$^3$/h |
| Wärmeleistung Druckdampf f | 112 MW |
| Stromproduktion Entspannungsturbine 6 | 36 MW |
| Abgeführte Leistung in Kühler 7 | 44 MW |
| Wärmeleistung abgekühltes Verbrennungsabgas g | 33 MW |
| Mengenstrom abgekühltes Verbrennungsabgas g | 640.000 Nm$^3$/h |
| Temperatur abgekühltes Verbrennungsabgas g | 128 °C |
| Elektrischer Gesamtwirkungsgrad | 0,56 |

| Tabelle 2: Autarker befeuerter Reaktor 2 (Figur 2) | |
|---|---|
| Mengenstrom Verbrennungsunterstützungsgas d | 1.067.000 Nm$^3$/h |
| Feuerungsleistung Brennstoff e | 1.000 MW |
| Strahlungszonenwirkungsgrad Strahlungszone 21 | 0,42 |
| Wärmeleistung Druckdampf f | 512 MW |
| Mengenstrom Druckdampf f | 595 t/h |
| Wärmeleistung abgekühltes Verbrennungsabgas g | 60 MW |
| Mengenstrom abgekühltes Verbrennungsabgas g | 1.172.000 Nm$^3$/h |
| Temperatur abgekühltes Verbrennungsabgas g | 128 °C |

| Tabelle 3: Kombinierte Anlage 400 (Figur 3) | |
|---|---|
| Stromproduktion Gasturbine 1 | 118 MW |
| Mengenstrom Verbrennungsunterstützungsgas a | 1.132.000 Nm$^3$/h |
| Feuerungsleistung Brennstoff b | 348 MW |
| Wärmeleistung Verbrennungsabgas c | 224 MW |
| Mengenstrom Verbrennungsabgas c | 1.170.000 Nm$^3$/h |
| Mengenstrom Verbrennungsunterstützungsgas d | 189.000 Nm$^3$/h |
| Feuerungsleistung Brennstoff e | 922 MW |
| Strahlungszonenwirkungsgrad Strahlungszone 21 | 0,36 |
| Wärmeleistung Druckdampf f | 650 MW |
| Mengenstrom Druckdampf f | 756 t/h |
| Wärmeleistung abgekühltes Verbrennungsabgas g | 69 MW |
| Mengenstrom abgekühltes Verbrennungsabgas g | 1.457.000 Nm$^3$/h |
| Temperatur abgekühltes Verbrennungsabgas g | 128 °C |

EP 3 189 223 B1

| Tabelle 4A: Anlage 100, Ausführungsform der Erfindung (Figur 4), 286 °C | |
|---|---|
| Stromproduktion Gasturbine 1 | 40 MW |
| Mengenstrom Verbrennungsunterstützungsgas a | 383.000 Nm$^3$/h |
| Feuerungsleistung Brennstoff b | 118 MW |
| Wärmeleistung Verbrennungsabgas c | 76 MW |
| Mengenstrom Verbrennungsabgas c | 395.000 Nm$^3$/h |
| Temperatur Verbrennungsabgas c | 656 °C |
| In Vorwärmeinrichtung 4 übertragen | 66 MW |
| Erwärmung Verbrennungsunterstützungsgas d von | 28 °C |
| Erwärmung Verbrennungsunterstützungsgas d auf | 286 °C |
| Mengenstrom Verbrennungsunterstützungsgas d | 879.000 Nm$^3$/h |
| Feuerungsleistung Brennstoff e | 824 MW |
| Strahlungszonenwirkungsgrad Strahlungszone 21 | 0,47 |
| Wärmeleistung Druckdampf f | 408 MW |
| Mengenstrom Druckdampf f | 475 t/h |
| Wärmeleistung abgekühltes Verbrennungsabgas g | 69 MW |
| Mengenstrom abgekühltes Verbrennungsabgas g | 966.000 Nm$^3$/h |
| Temperatur abgekühltes Verbrennungsabgas g | 128 °C |

| Tabelle 4B: Anlage 100, Ausführungsform der Erfindung (Figur 4), 498 °C | |
|---|---|
| Stromproduktion Gasturbine 1 | 60 MW |
| Mengenstrom Verbrennungsunterstützungsgas a | 580.000 Nm$^3$/h |
| Wärmeleistung Verbrennungsabgas c | 115 MW |
| Mengenstrom Verbrennungsabgas c | 599.000 Nm$^3$/h |
| Temperatur Verbrennungsabgas c | 656 °C |
| In Vorwärmeinrichtung 4 übertragen | 100 MW |
| Erwärmung Verbrennungsunterstützungsgas d von | 28 °C |
| Erwärmung Verbrennungsunterstützungsgas d auf | 498 °C |
| Mengenstrom Verbrennungsunterstützungsgas d | 765.000 Nm$^3$/h |
| Feuerungsleistung Brennstoff e | 717 MW |
| Strahlungszonenwirkungsgrad Strahlungszone 21 | 0,51 |
| Wärmeleistung Druckdampf f | 335 MW |
| Mengenstrom Druckdampf f | 390 t/h |
| Wärmeleistung abgekühltes Verbrennungsabgas g | 50 MW |
| Temperatur abgekühltes Verbrennungsabgas g | 128 °C |

| Tabelle 5: Anlage 200, Ausführungsform der Erfindung (Figur 5) | |
|---|---|
| Stromproduktion Gasturbine 1 | 108 MW |

(fortgesetzt)

| Tabelle 5: Anlage 200, Ausführungsform der Erfindung (Figur 5) | |
|---|---|
| Mengenstrom Verbrennungsunterstützungsgas a | 1.035.000 Nm$^3$/h |
| Feuerungsleistung Brennstoff b | 318 MW |
| Wärmeleistung Verbrennungsabgas c | 211 MW |
| Mengenstrom Verbrennungsabgas c | 765.000 Nm$^3$/h |
| Temperatur Verbrennungsabgas c | 656 °C |
| In Vorwärmeinrichtung 4 übertragene Energie | 77 MW |
| Erwärmung Verbrennungsunterstützungsgas d von | 28 °C |
| Erwärmung Verbrennungsunterstützungsgas d auf | 627 °C |
| Feuerungsleistung Brennstoff e | 799 MW |
| Strahlungszonenwirkungsgrad Strahlungszone 21 | 0,42 |
| Wärmeleistung Druckdampf f | 512 MW |
| Mengenstrom Druckdampf f | 595 t/h |
| Wärmeleistung abgekühltes Verbrennungsabgas g | 60 MW |
| Mengenstrom abgekühltes Verbrennungsabgas g | 1.172.000 Nm$^3$/h |
| Temperatur abgekühltes Verbrennungsabgas g | 128 °C |

Die nachfolgenden Tabellen 6A bis 6C stellen vergleichbare Verfahren zur Veranschaulichung der Vorteile der vorliegenden Erfindung einander gegenüber. In allen Tabellen soll jeweils die gleiche Strommenge (Tabellenzeile "Stromproduktion") in der Anlage insgesamt und eine gleiche Menge eines Reaktionsprodukts (hier des Kohlenwasserstoffs Ethylen, Tabellenzeile "Ethylenproduktion" in einem Reaktor 2 erzeugt werden. Es wird explizit auf die obigen Figuren und insbesondere die einleitenden Absätze der Figurenbeschreibung Bezug genommen.

Ferner wird hier davon ausgegangen, dass mit dem Druckdampf f jeweils Strom erzeugt wird. Dies dient vorrangig der besseren Vergleichbarkeit der Prozesse hinsichtlich des elektrischen Wirkungsgrads und der "Effizienz" im Sinne der obigen Definitionen. Die Tabellenzeile "Stromproduktion" bzw. die in dieser angegebenen Werte umfassen daher auch dem aus dem Druckdampf f erzeugten Strom, wobei ein für Druckdampf f mit 520 °C und 120 bar typischer elektrischer Wirkungsgrad von 0,24 angenommen wird.

Die Tabellenspalte 1 ("Reaktor, Netzstrom") enthält jeweils Werte für Strom, der aus einem Stromnetz bereitgestellt wird, und für einen autark betriebenen Reaktor 2 gemäß Figur 2. Für die Erzeugung des aus dem Stromnetz bereitgestellten Stroms wird ein Wirkungsgrad von 0,33 angenommen. Dies entspricht einer typischen Bewertungszahl für Strom aus üblichen Stromnetzen (d.h. dem Mittelwert des elektrischen Wirkungsgrads über einen Kraftwerkspark eines Versorgers aus neuen und alten Kraftwerken mit beliebigen Kraftwerksarten, d.h. reinen (Kohle-)Dampfkraftwerken und Gas-und-Dampf-Kraftwerken, sowie inklusive sämtlicher Leitungsverluste). Der Strom wird also gemäß Spalte 1 aus dem Netz zur Verfügung gestellt, soweit er nicht aus dem Druckdampf f erzeugt wird.

Die dem Wirkungsgrad 0,33 entsprechende Feuerungsleistung, die zur Erzeugung dieses aus dem Stromnetz bereitgestellten Stromanteils erforderlich wäre, geht zur Vergleichbarkeit der Verfahren zusätzlich zu der Heizleistung für den Reaktor 2 in die gesamte erforderliche Heizleistung (Tabellenzeile "Heizleistung gesamt") ein. Diese Heizleistung ist zusätzlich als auf die in Tabellenspalte 2 angegebene Heizleistung normierte Heizleistung (Tabellenzeile "Heizleistung %") angegeben.

Die Tabellenspalte 2 ("Reaktor, Gas-und-Dampf-Kraftwerk") gibt Werte für eine Kombination aus einem separaten Gas-und-Dampf-Kraftwerk, beispielsweise gemäß Figur 1, und einem, z.B. bereits in Tabellenspalte 1 angegebenen, autark betriebenen Reaktor 2 gemäß Figur 2 an. Die für die Stromproduktion in dem Gas-und-Dampf-Kraftwerk erforderliche Heizleistung richtet sich nach dem hier angenommenen elektrischen Gesamtwirkungsgrad von 0,56 (siehe Erläuterungen zu Figur 1) und geht in die in den genannten Tabellenzeilen angegebene gesamte Heizleistung (zusätzlich zur Heizleistung für den Reaktor 2) ein.

Aus der Zusammenschau der Tabellenspalten 1 und 2 ist ersichtlich, dass bei gleicher erzeugter elektrischer Leistung (Tabellenzeile "Stromproduktion"), die aus dem Stromnetz entnommenen (Tabellenspalte 1) bzw. in dem Gas-und-Dampf-Kraftwerk erzeugten (Tabellenspalte 2) Strom umfasst, und einer gleichen Produktionsmenge des Reaktionsprodukts Ethylen schon aufgrund der unterschiedlichen elektrischen Wirkungsgrade (0,56 elektrischer Gesamtwirkungsgrad bei Stromerzeugung in einem Gas-und-Dampf-Kraftwerk, siehe Erläuterungen zu Figur 1; 0,33 für typischen Netzstrom, siehe oben) gemäß Tabellenspalte 2 eine Verringerung der erforderlichen Heizleistung erzielbar ist.

Der spezifische Energieverbrauch des Verfahrens, bezogen auf dieselbe Menge des Reaktionsprodukts Ethylen (Tabellenzeile "Spezif. Energieverbrauch") verringert sich entsprechend, so dass sich die "energetische Effizienz" des Verfahrens im eingangs erläuterten Sinn entsprechend erhöht. Die den Reaktor 2 betreffenden Wirkungsgrade ändern sich nicht, da der Reaktor 2 weiterhin autark betrieben wird.

In der Tabelle 6A sind den Werten der soeben erläuterten Tabellenspalten 1 und 2 in der Tabellenspalte 3 ("Kombinierte Anlage 400 gemäß Figur 3") Werte bezüglich einer kombinierten Anlage 400 gemäß Figur 3 bei einer einer auch hier mit 152 MW angenommenen gesamten Stromproduktion gegenübergestellt. Von diesen 152 werden gemäß Tabellenspalte 3 118 MW durch die Gasturbine 1 erzeugt und 34 MW als aus dem Druckdampf f bei entsprechend (niedrigem) Wirkungsgrad erzeugt angenommen. (Diese Vereinfachung dient der Vergleichbarkeit, auch wenn die Dampfnutzung des Druckdampfs f in der Praxis typischerweise die direkte Nutzung der Wellenleistung zum Antrieb von Verdichtern oder Pumpen ist - was allerdings bis auf einen marginalen Verlust am Generator von typischerweise etwa 1% der elektrischen Leistung entspicht).

Aufgrund des von ca. 0,42 auf ca. 0,37 verschlechterten Strahlungszonenwirkungsgrads muss in diesem Fall für dieselbe Menge an Reaktionsprodukten eine erhöhte Heizleistung in dem Reaktor 2 eingesetzt werden. Die Heizleistung beträgt aus diesem Grund gemäß Tabellenspalte 3 insgesamt 1.270 MW, was zwar eine deutliche Verbesserung gegenüber der Tabellenspalte 1, jedoch nur eine marginale Verbesserung gegenüber Tabellenspalte 2 von 0,1% darstellt.

| Tabelle 6A | | Reaktor, Netzstrom | Reaktor, Gas-und-Dampf-Kraftwerk | Kombinierte Anlage 400 gemäß Figur 3 |
|---|---|---|---|---|
| | | 1 | 2 | 3 |
| Parameter | Einheit | | | |
| Ethylenproduktion | t/h | 176 | 176 | 176 |
| Stromproduktion | MW | 152 | 152 | 152 |
| Spezif. Energieverbrauch Spezif. Energieverbrauch % | Gcal/t | 6,85 | 5,93 | 5,93 |
| | | 115,4% | 100,0% | 99,9% |
| Heizleistung gesamt Heizleistung % | MW | 1.459 | 1.272 | 1.270 |
| | | 114,7% | 100,0% | 99,9% |

Der Brennstoffverbrauch zur Erzeugung elektrischer Energie wird in den beiden Benchmarkfällen gemäß Spalte 1 und 2 (d.h. bei getrennter und jeweils autarker Erzeugung von Strom und Reaktionsprodukten) jeweils für die bei gekoppelter Produktion mögliche elektrische Leistung bestimmt. (Der Benchmark-Fall wird also jeweis "angepasst", wohlwissend, dass Gas-und-Dampf-Kraftwerke mit weniger als 80 MW elektrischer Leistung kaum als eigenständiges Kraftwerk errichtet werden. Dies gilt auch für die nachfolgenden Tabellen.) Heizleistung und spezifischer Energieverbrauch korrelieren miteinander, da der Heizbedarf deutlich über die Hälfte des gesammten Energieverbrauchs ausmachen, d.h.

In der Tabelle 6B sind den Werten der bereits zu Tabelle 6A erläuterten Tabellenspalten 1 und 2, deren Bedeutung gleich jenen der Tabelle 6A ist, in der Tabellenspalte 3 ("Kombinierte Anlage gemäß Figur 5") Werte bezüglich einer kombinierten Anlage 200 gemäß Figur 5 bei einer angenommenen Stromproduktion von 108 MW gegenübergestellt. Es handelt sich hierbei um die Stromproduktion der Gasturbine 1, da die Dampfproduktion in dem Reaktor 2 gleich der Dampfproduktion in einem autarken Reaktor ist.

Aufgrund der Aufteilung des Verbrennungsabgases c auf die Teilströme c' und c'' und der teilweisen Verwendung nur zur Vorwärmung in der Vorwärmeinrichtung 4 und der teilweisen Einspeisung in den Reaktor 2 kann in einer kombinierten Anlage 200 gemäß Figur 5 der Strahlungszonenwirkungsgrad hier gegenüber den Tabellenspalten 1 und 2 konstant gehalten werden, nämlich auf dem oben erwähnten Wert von ca. 0,42. Bei der spezifischen angegebenen Parameterkombination reduziert sich daher die erforderliche Heizleistung deutlich, d.h. gegenüber Tabellenspalte 2 um ca. 6% und gegenüber Tabellenspalte 1 um ca. 17%. Entsprechend sinkt auch der spezifische Energieverbrauch, bezogen auf dieselbe Menge des Reaktionsprodukts Ethylen deutlich. Gleichzeitig kann, wie erwähnt, ein entsprechender Reaktor 2 weiter bei herkömmlichen Bedingungen betrieben werden.

| Tabelle 6B | | Reaktor, Netzstrom | Reaktor, Gas-und- Dampf-Kraftwerk | Kombinierte Anlage 200 gemäß Figur 5 |
|---|---|---|---|---|
| | | 1 | 2 | 3 |
| Parameter | Einheit | | | |
| Ethylenproduktion | t/h | 176 | 176 | 176 |
| Stromproduktion | MW | 108 | 108 | 108 |
| Spezif. Energieverbrauch Spezif. Energieverbrauch % | Gcal/t | 6,58 | 5,93 | 5,56 |
| | | 111,0% | 100,0% | 93,8% |
| Heizleistung gesamt Heizleistung % | MW | 1.327 | 1.193 | 1.118 |
| | | 111,2% | 100,0% | 93,7% |

In der Tabelle 6C sind den Werten der erläuterten Tabellenspalten 1 und 2 in der Tabellenspalte 3 ("Kombinierte Anlage gemäß Figur 4, 498 °C") Werte bezüglich einer kombinierten Anlage 100 gemäß Figur 4 bei einer Vorwärmung des Verbrennungsunterstützungsgases d auf 498 °C, d.h. entsprechend Tabelle 4B, und einer angenommenen Stromproduktion durch die Gasturbine von 60 MW gegenübergestellt. Von diesen 60 MW müssen 43 MW abgezogen werden (Tabellenzeile " Verminderte Dampfproduktion als el. Leistung"), da weniger Dampf produziert wird und die entsprechende fehlende Wellenleistung vereinfacht durch elektrische Leistung kompensiert wird. Verluste an einem verwendeten Elektromotor von typischerweise ca. 3% werden der Einfachheit halber vernachlässigt

Aufgrund des hier nochmals drastisch erhöhten Strahlungszonenwirkungsgrads, nämlich auf einen Wert von ca. 0,53, kommt es hier zu einer nochmaligen deutlichen Reduktion der Heizleistung bei gegenüber den Tabellenspalten 1 und 2 identischer Stromproduktion.

| Tabelle 6C | | Reaktor, Netzstrom | Reaktor, Gas-und- Dampf-Kraftwerk | Kombinierte Anlage 100 gemäß Figur 4, 498 °C |
|---|---|---|---|---|
| | | 1 | 2 | 3 |
| Parameter | Einheit | | | |
| Ethylenproduktion | t/h | 176 | 176 | 176 |
| Stromproduktion | MW | 60 | 60 | 60 |
| Spezif. Energieverbrauch | Gcal/t | 6,03 | 5,93 | 5,27 |
| Spezif. Energieverbrauch % | | 101,7% | 100,0% | 88,8% |
| Heizleistung gesamt | MW | 1.052 | 1.031 | 896 |
| Heizleistung % | | 102,0% | 100,0% | 86,90% |
| Verminderte Dampfproduktion als el. Leistung | MW | 0 | 0 | 43 |
| Stromproduktion netto | MW | 17 | 17 | 17 |

Man erkennt aus der Zusammenschau der obigen Tabellen insbesondere, dass die teilweise Verstromung eines (hochwertigen) Brennstoffs, beispielsweise eines Restgases aus einem entsprechenden Verfahren, das mittels des Reaktors 2 realisiert wird, gegenüber einer Erzeugung von Strom über einen typischen Kraftwerksmix bzw. einer Entnahme aus dem Netz eine Effizienzsteigerung von etwa 11% bewirkt, unabhängig davon, ob ein Gas-und-Dampf-Kraftwerk gemäß Figur 1 oder eine bekannte Kombination in Form einer Anlage 400 gemäß Figur 3 eingesetzt wird. Voraussetzung ist hierbei insbesondere die ausreichende Verfügbarkeit beispielsweise eines entsprechenden Restgases.

Ferner erkennt man, dass die bekannte Kombination in Form einer Anlage 400 gemäß Figur 3 gegenüber einer getrennten Erzeugung von Strom und von Reaktionsprodukten keinen bzw. nur einen geringen Effizienzvorteil bietet, d.h. in den oben gezeigten Beispielen eine gleiche Heizleistung und damit ein vergleichbarer Brennstoffbedarf erforderlich sind.

Die gemäß einer Ausführungsform der Erfindung vorgeschlagene Anlage 200 hat demgegenüber eine etwa 6% höhere Effizienz gegenüber einer separaten Stromerzeugung mittels eines Gas-und-Dampf-Kraftwerks und der separaten Erzeugung der Reaktionsprodukte in einem autarken Reaktor 2. Gegenüber einem typischen Kraftwerksmix bzw. einer Entnahme von Strom aus dem Netz und der separaten Erzeugung der Reaktionsprodukte in einem autarken Reaktor 2 ist eine Effizienzsteigerung von etwa 11% zu beobachten.

Die Anlage 200 erzeugt pro zusätzlich eingesetzter Einheit Feuerungsleistung 92% Strom (d.h. pro 1 MW Heizleistung, die zusätzlich zu der für den autarken Reaktorbetrieb erforderlichen Heizleistung eingesetzt werden, werden 0,92 MW Strom erzeugt). Dies entspricht einem fast zweifachen elektrischen Wirkungsgrad einer eines Gas-und-Dampf-Kraftwerks bzw. dem dreifachen elektrischen Wirkungsgrad gegenüber einem Kraftwerksmix bzw. der Entnahme von Strom aus dem Netz.

Die vorgeschlage Anlage 100 braucht weniger Heizleistung als der autarke Reaktor und produziert noch zusätzlich Strom. Die Anlage 100 hat damit eine etwa 11% höhere Effizienz gegenüber einer separaten Stromerzeugung mittels eines Gas-und-Dampf-Kraftwerks und der separaten Erzeugung der Reaktionsprodukte in einem autarken Reaktor 2.

**Patentansprüche**

1. Verfahren zur kombinierten Erzeugung von mechanischer Leistung und Herstellung von Kohlenwasserstoffen, bei dem zur Erzeugung der mechanischen Leistung wenigstens eine Verbrennungskraftmaschine (1) unter Erhalt eines Verbrennungsabgases (c) befeuert wird und bei dem zur Herstellung der Kohlenwasserstoffe wenigstens ein Reaktor (2) unter Verwendung eines Brennstoffs (e) und eines Verbrennungsunterstützungsgases (d) beheizt wird, **dadurch gekennzeichnet, dass** zumindest ein Teil des Verbrennungsunterstützungsgases (d) durch indirekten Wärmetausch mit zumindest einem Teil des Verbrennungsabgases (c) der Verbrennungskraftmaschine (1) erwärmt wird.

2. Verfahren nach Anspruch 1, bei dem die mechanische Leistung zumindest teilweise mittels wenigstens eines Generators (G) in elektrische Leistung umgewandelt und/oder zum Antreiben wenigstens einer Welle verwendet wird.

3. Verfahren nach Anspruch 1 oder 2, bei dem der wenigstens eine Reaktor (2) als Rohrreaktor ausgebildet ist, in dem in einer Strahlungszone Reaktionsrohre von außen durch Brenner beheizt werden, in denen der Brennstoff verbrannt wird.

4. Verfahren nach Anspruch 3, bei dem zur Herstellung der Kohlenwasserstoffe ein Einsatz mit Dampf unter Druck durch die Reaktionsrohre des als Rohrreaktor ausgebildeten Reaktors (2) geführt wird.

5. Verfahren nach Anspruch 3 oder 4, bei dem in den Reaktionsrohren des als Rohrreaktor ausgebildeten Reaktors (2) ein Katalysator bereitgestellt ist.

6. Verfahren nach einem der vorstehenden Ansprüche, bei dem der Brennstoff (e) zumindest teilweise aus einem Gasgemisch gebildet wird, das aus Produktstrom des wenigstens einen Reaktors (2) abgetrennt wird.

7. Verfahren nach einem der vorstehenden Ansprüche, bei dem zumindest ein Bereich des wenigstens einen Reaktors (2) durch die Beheizung unter Verwendung des Brennstoffs (e) und des Verbrennungsunterstützungsgases (d) auf ein Temperaturniveau von 1.500 bis 2.500 °C, insbesondere auf ein Temperaturniveau von 1.500 bis 2.000 °C, aufgeheizt wird.

8. Verfahren nach einem der vorstehenden Ansprüche, bei dem die wenigstens eine Verbrennungskraftmaschine (1) wenigstens eine Gasturbine umfasst.

9. Verfahren nach einem der vorstehenden Ansprüche, bei dem das Verbrennungsabgas (c) der Verbrennungskraftmaschine (1) auf einem Temperaturniveau von 500 bis 1.000 °C, insbesondere auf einem Temperaturniveau von 600 bis 700 °C oder auf einem Temperaturniveau von 500 bis 650 °C, bereitgestellt wird.

10. Verfahren nach einem der vorstehenden Ansprüche, bei dem ein Teil des Verbrennungsabgases (c) der Verbrennungskraftmaschine (1) zur Erwärmung des Verbrennungsunterstützungsgases (d) durch indirekten Wärmetausch verwendet und ein Teil des Verbrennungsabgases (c) der Verbrennungskraftmaschine (1) mit dem Verbrennungsunterstützungsgas (d) vereinigt und zusammen mit diesem dem wenigstens einen Reaktor (2) zugeführt wird.

11. Verfahren nach einem der Ansprüche 1 bis 9, bei dem das Verbrennungsabgas (c) der Verbrennungskraftmaschine (1) vollständig zur Erwärmung des Verbrennungsunterstützungsgases (d) durch indirekten Wärmetausch verwendet und nicht dem wenigstens einen Reaktor (2) zugeführt wird.

12. Verfahren nach einem der vorstehenden Ansprüche, bei dem Erdgas und/oder ein methanhaltiges Gasgemisch, insbesondere ein gemäß Anspruch 6 gebildetes Gasgemisch, das Wasserstoff, Methan und Kohlenmonoxid enthält, als der Brennstoff (e) und/oder Luft als das Verbrennungsunterstützungsgas (d) verwendet wird.

13. Verfahren nach einem der vorstehenden Ansprüche, bei dem aus Abwärme des wenigstens einen Reaktors (2) Druckdampf (f) erzeugt und zum Antrieb wenigstens einer Welle, insbesondere eines Generators (G), verwendet wird.

14. Anlage (100, 200) zur kombinierten Erzeugung von mechanischer Leistung und Herstellung von Kohlenwasserstoffen, die zur Erzeugung der mechanischen Leistung wenigstens eine Verbrennungskraftmaschine (1) umfasst, die unter Erhalt eines Verbrennungsabgases (c) befeuerbar ist, und die zur Herstellung der Kohlenwasserstoffe wenigstens einen Reaktor (2) umfasst, der unter Verwendung eines Brennstoffs (e) und eines Verbrennungsunterstützungsgases (d) beheizbar ist, **dadurch gekennzeichnet, dass** Mittel vorgesehen sind, die dafür eingerichtet sind, zumindest ein Teil des Verbrennungsunterstützungsgases (d) durch indirekten Wärmetausch mit zumindest einem Teil des Verbrennungsabgases (c) der Verbrennungskraftmaschine (1) zu erwärmen.

15. Anlage (100, 200) nach Anspruch 14, die zur Durchführung eines Verfahrens nach einem der Ansprüche 1 bis 13 eingerichtet ist.

**Claims**

1. Method for the combined generation of mechanical power and production of hydrocarbons, in which in order to generate the mechanical power at least one internal combustion engine (1) is fired up, thereby producing a combustion exhaust gas (c), and in which in order to produce the hydrocarbons at least one reactor (2) is heated using a fuel (e) and a combustion-supporting gas (d), **characterized in that** at least part of the combustion-supporting gas (d) is heated by indirect heat exchange with at least part of the combustion exhaust gas (c) from the internal combustion engine (1).

2. Method according to Claim 1, in which the mechanical power is at least partially converted into electrical power by means of at least one generator (G) and/or is used for driving at least one shaft.

3. Method according to Claim 1 or 2, in which the at least one reactor (2) is formed as a tubular reactor in which, in a radiation zone, reaction tubes are heated from outside by burners in which the fuel is burned.

4. Method according to Claim 3, in which in order to produce the hydrocarbons a feed is passed through the reaction tubes of the reactor (2) formed as a tubular reactor by means of pressurized steam.

5. Method according to Claim 3 or 4, in which a catalyst is provided in the reaction tubes of the reactor (2) formed as a tubular reactor.

6. Method according to one of the preceding claims, in which the fuel (e) is at least partially formed from a gas mixture which is separated from a product stream from the at least one reactor (2).

7. Method according to one of the preceding claims, in which at least one region of the at least one reactor (2) is heated up to a temperature level of 1500 to 2500°C, in particular to a temperature level of 1500 to 2500°C, by the heating using the fuel (e) and the combustion-supporting gas (d).

8. Method according to one of the preceding claims, in which the at least one internal combustion engine (1) comprises at least one gas turbine.

9. Method according to one of the preceding claims, in which the combustion exhaust gas (c) from the internal combustion engine (1) is provided at a temperature level of 500 to 1000°C, in particular at a temperature level of 600 to 700°C or at a temperature level of 500 to 650°C.

10. Method according to one of the preceding claims, in which part of the combustion exhaust gas (c) from the internal combustion engine (1) is used for heating the combustion-supporting gas (d) by indirect heat exchange and part of the combustion exhaust gas (c) from the internal combustion engine (1) is combined with the combustion-supporting gas (d) and supplied together with it to the at least one reactor (2).

11. Method according to one of Claims 1 to 9, in which the combustion exhaust gas (c) from the internal combustion engine (1) is used in its entirety for heating the combustion-supporting gas (d) by indirect heat exchange and is not supplied to the at least one reactor (2).

12. Method according to one of the preceding claims, in which natural gas and/or a methane-containing gas mixture, in particular a gas mixture formed according to Claim 6 which contains hydrogen, methane and carbon monoxide, is used as the fuel (e) and/or air is used as the combustion-supporting gas (d).

13. Method according to one of the preceding claims, in which pressurized steam (f) is produced from waste heat from the at least one reactor (2) and is used for driving at least one shaft, in particular of a generator (G).

14. System (100, 200) for the combined generation of mechanical power and production of hydrocarbons, which comprises for the generation of the mechanical power at least one internal combustion engine (1) which can be fired up, thereby producing a combustion exhaust gas (c), and which comprises for the production of the hydrocarbons at least one reactor (2) which can be heated using a fuel (e) and a combustion-supporting gas (d), **characterized in that** means designed to heat at least part of the combustion-supporting gas (d) by indirect heat exchange with at least part of the combustion exhaust gas (c) from the internal combustion engine (1) are provided.

15. System (100, 200) according to Claim 14, which is designed for carrying out a method according to one of Claims 1 to 13.

**Revendications**

1. Procédé pour, en combinaison, générer de la puissance mécanique et produire des hydrocarbures, dans lequel, pour la génération de la puissance mécanique, au moins un moteur à combustion interne (1) est alimenté avec obtention d'un gaz d'échappement de combustion (c) et dans lequel, pour la production des hydrocarbures, au moins un réacteur (2) est chauffé en utilisant un combustible (e) et un gaz de support de combustion (d), **caractérisé en ce qu'**au moins une partie du gaz de support de combustion (d) est chauffée par échange thermique indirect avec au moins une partie du gaz d'échappement de combustion (c) du moteur à combustion interne (1).

2. Procédé selon la revendication 1, dans lequel la puissance mécanique est convertie au moins en partie au moyen d'au moins un générateur (G) en puissance électrique et/ou est utilisée pour entraîner au moins un arbre.

3. Procédé selon la revendication 1 ou 2, dans lequel l'au moins un réacteur (2) est réalisé sous forme de réacteur tubulaire dans lequel, dans une zone de rayonnement, des tubes de réaction sont chauffés depuis l'extérieur par des brûleurs dans lesquels est brûlé le combustible.

4. Procédé selon la revendication 3, dans lequel, pour la production des hydrocarbures, une charge est guidée avec de la vapeur sous pression à travers les tubes de réaction du réacteur (2) réalisé sous forme de réacteur tubulaire.

5. Procédé selon la revendication 3 ou 4, dans lequel, dans les tubes de réaction du réacteur (2) réalisé sous forme de réacteur tubulaire est prévu un catalyseur.

6. Procédé selon l'une quelconque des revendications précédentes, dans lequel le combustible (e) est formé au moins en partie d'un mélange de gaz qui est séparé à partir du flux de produit de l'au moins un réacteur (2).

7. Procédé selon l'une quelconque des revendications précédentes, dans lequel au moins une région de l'au moins un réacteur (2) est chauffée à un niveau de température de 1500 à 2500°C, en particulier à un niveau de température de 1500 à 2000°C par le chauffage en utilisant le combustible (e) et le gaz de support de combustion (d) .

8. Procédé selon l'une quelconque des revendications précédentes, dans lequel l'au moins un moteur à combustion interne (1) comprend au moins une turbine à gaz.

9. Procédé selon l'une quelconque des revendications précédentes, dans lequel le gaz d'échappement de combustion (c) du moteur à combustion interne (1) est fourni à un niveau de température de 500 à 1000°C, en particulier à un niveau de température de 600 à 700°C ou à un niveau de température de 500 à 650°C.

10. Procédé selon l'une quelconque des revendications précédentes, dans lequel une partie du gaz d'échappement de combustion (c) du moteur à combustion interne (1) est utilisée pour chauffer le gaz de support de combustion (d) par échange thermique indirect et une partie du gaz d'échappement de combustion (c) du moteur à combustion interne (1) est réunie au gaz de support de combustion (d) et est acheminée conjointement avec celui-ci à l'au moins un réacteur (2).

11. Procédé selon l'une quelconque des revendications 1 à 9, dans lequel le gaz d'échappement de combustion (c) du moteur à combustion interne (1) est utilisé complètement pour chauffer le gaz de support de combustion (d) par échange thermique indirect et n'est pas acheminé à l'au moins un réacteur (2).

12. Procédé selon l'une quelconque des revendications précédentes, dans lequel du gaz naturel et/ou un mélange de gaz contenant du méthane, en particulier un mélange de gaz formé selon la revendication 6, qui contient de l'hydrogène, du méthane et du monoxyde de carbone, est utilisé en tant que combustible (e) et/ou de l'air est utilisé en tant que gaz de support de combustion (d).

13. Procédé selon l'une quelconque des revendications précédentes, dans lequel à partir de la chaleur perdue de l'au moins un réacteur (2), de la vapeur sous pression (f) est produite et est utilisée pour entraîner au moins un arbre, en particulier un générateur (G).

14. Installation (100, 200) pour, en combinaison, générer de la puissance mécanique et produire des hydrocarbures, qui comprend, pour la génération de la puissance mécanique, au moins un moteur à combustion interne (1) qui peut être alimenté avec obtention d'un gaz d'échappement de combustion (c) et qui comprend, pour la production des hydrocarbures, au moins un réacteur (2) qui peut être chauffé en utilisant un combustible (e) et un gaz de support de combustion (d), **caractérisée en ce que** des moyens sont prévus qui sont adaptés pour chauffer au moins une partie du gaz de support de combustion (d) par échange thermique indirect avec au moins une partie du gaz d'échappement de combustion (c) du moteur à combustion interne (1).

15. Installation (100, 200) selon la revendication 14, qui est prévue pour mettre en oeuvre un procédé selon l'une quelconque des revendications 1 à 13.

# Fig. 1

# Fig. 2

**Fig. 3**

**Fig. 4**

**Fig. 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2148734 A
- US 5048284 A
- GB 2296719 A
- FR 1445870 A

**In der Beschreibung aufgeführte Nicht-Patentliteratur**

- Ethylene'' in Ullmann's Encyclopedia of Industrial Chemistry. 15. April 2007
- Propene. Ullmann's Encyclopedia of Industrial Chemistry, Onlineausgabe. 15. Juni 2000